(19) Europäisches Patentamt
European Patent Office
Office européen des brevets

(11) EP 1 853 920 B1

(12) EUROPEAN PATENT SPECIFICATION

(45) Date of publication and mention of the grant of the patent:
**01.02.2012 Bulletin 2012/05**

(21) Application number: **06736983.5**

(22) Date of filing: **03.03.2006**

(51) Int Cl.:
***G01N 33/58*** *(2006.01)*

(86) International application number:
**PCT/US2006/007748**

(87) International publication number:
**WO 2006/094239 (08.09.2006 Gazette 2006/36)**

(54) **FLUORESCENCE POLARIZATION ASSAYS FOR ACETYLTRANSFERASE/DEACETYLASE ACTIVITY**

FLUORESZENZPOLARISATIONSTESTS FÜR DIE ACETYLTRANSFERASE-DEACETYLASE-AKTIVITÄT

DOSAGES PAR POLARISATION DE FLUORESCENCE SERVANT A DÉTERMINER L'ACTIVITÉ ACETYLTRANSFÉRASE/DÉACETYLASE

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.03.2005 US 658711 P**
**04.08.2005 US 705609 P**
**02.12.2005 US 741783 P**

(43) Date of publication of application:
**14.11.2007 Bulletin 2007/46**

(73) Proprietor: **Sirtris Pharmaceuticals, Inc.**
**Cambridge, MA 02139 (US)**

(72) Inventors:
- **MILNE, Jill**
**Brookline, Massachusetts 02446-2768 (US)**
- **CARNEY, David**
**Derry, New Hampshire 03038 (US)**

(74) Representative: **Chapman, Paul Gilmour et al**
**Marks & Clerk LLP**
**Aurora**
**120 Bothwell Street**
**Glasgow G2 7JS (GB)**

(56) References cited:
**WO-A2-02/14543**

- **WEGENER DENNIS ET AL: "Improved fluorogenic histone deacetylase assay for high-throughput-screening applications." ANALYTICAL BIOCHEMISTRY, vol. 321, no. 2, 15 October 2003 (2003-10-15), pages 202-208, XP002402578 ISSN: 0003-2697**
- **NIKIFOROV T T ET AL: "APPLICATION OF FLUORESCENCE POLARIZATION TO ENZYME ASSAYS AND SINGLE NUCLEOTIDE POLYMORPHISM GENOTYPING: SOME RECENT DEVELOPMENTS" CURRENT TOPICS IN MEDICINAL CHEMISTRY, BENTHAM SCIENCE PUBLISHERS, HILVERSUM, NL, vol. 6, no. 3, 2003, pages 201-212, XP008064627 ISSN: 1568-0266**
- **LEVINE LEANNA M ET AL: "Measurement of specific protease activity utilizing fluorescence polarization" ANALYTICAL BIOCHEMISTRY, vol. 247, no. 1, 1997, pages 83-88, XP002402579 ISSN: 0003-2697**
- **BONIN PAUL D ET AL: "Development of a fluorescence polarization assay for peptidyl-tRNA hydrolase" ANALYTICAL BIOCHEMISTRY, vol. 306, no. 1, 1 July 2002 (2002-07-01), pages 8-16, XP002402580 ISSN: 0003-2697**
- **MOHAMMED SARWAR NASIR ET AL: "Fluorescence Polarization: an analytical tool for immunoassay and drug discovery" COMBINATORIAL CHEMISTRY AND HIGH THROUGHPUT SCREENING, HILVERSUM, NL, vol. 2, no. 4, 1999, pages 177-190, XP001062258 ISSN: 1386-2073**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DEVANE R ET AL: "A molecular dynamics method for calculating molecular volume changes appropriate for biomolecular simulation", BIOPHYSICAL JOURNAL BIOPHYS. SOC USA, vol. 85, no. 5, November 2003 (2003-11), pages 2801-2807, ISSN: 0006-3495
- D.E. STERNER AND S.L. BERGER: "Acetylation of Histones and Transcription-Related Factors", MICROBIOLOGY AND MOLECULAR BIOLOGY REVIEWS, vol. 64, no. 2, June 2000 (2000-06), pages 435-459,
- MARCOTTE PATRICK A ET AL: "Fluorescence assay of SIRT protein deacetylases using an acetylated peptide substrate and a secondary trypsin reaction", ANALYTICAL BIOCHEMISTRY, vol. 332, no. 1, 1 September 2004 (2004-09-01), pages 90-99, ISSN: 0003-2697

## Description

## BACKGROUND

Acetylation and deacetylation of histone proteins, transcription factors, and related proteins play a major role in the control of cellular processes. In particular, the acetylation state of histones controls how tightly the histone proteins interact with DNA, and therefore how accessible the DNA is to transcription factors. Enzymes that add acetyl groups to histones or other proteins are called histone acetyltransferases (HATs). Enzymes that remove the acetyl groups fall into two families: the histone deacetylases (HDACs) and the Sir2 family of deacetylases. Currently there are eleven known members of the mammalian HDAC family (Gray and Ekstrom, Exper. Cell Res. 2001, 262, 75-83; Zhou, et al. Proc. Natl. Acad. Sci. USA 2001, 98, 10572-10577; Kao et al. J. Biol. Chem. 2002, 277, 187-193; Gao et al. J. Biol. Chem. 2002, 277, 25748-25755) and seven members of the Sir2 family (Gray and Ekstrom, Exper. Cell Res. 2001, 262, 75-83).

Histone acetyltransferases catalyze the transfer of an acetyl group from acetyl-CoA to the ε-amino group of a lysine residue on the target protein. Many HAT enzymes have been characterized from eukaryotic organisms (Sterner and Berger, Microbiol. Mol. Biol. Rev. 2000, 64, 435-459). HDAC enzymes utilize a zinc ion at the active site of the protein to catalyze the removal of the acetyl group from acetyllysine in the form of acetate. Members of the Sir2 family of enzymes use NAD as a cofactor in the hydrolysis of acetyllysine.

The acetylation state of histone proteins plays a major role in gene expression and in cell-cycle control, and appears to play a role in certain forms of cancer. In particular, abnormal recruitment of histone deacetylases by corepressor proteins has been shown to promote the development of promyelocytic leukemia. In tumor cell lines, several studies have shown that treatment with HDAC inhibitors can lead to growth inhibition, growth arrest, terminal differentiation, and/or apoptosis. In vivo studies have demonstrated growth inhibition of tumors and a reduction in tumor metastasis as a result of treatment with HDAC inhibitors (Kramer et al. Trends Endocrinol. Metab. 2001, 12, 294-300).

Effective study of the enzymology and modulation of HATs, HDACs, and Sir2 enzymes depends on the availability of robust assays capable of being performed in a high-throughput manner. Several assay methodologies have been developed for these enzymes, with varying degrees of utility for inhibitor and activator screening.

Histone acetyltransferase assays are typically radioactivity-based. In these formats, acetyl-CoA radiolabeled on the acetyl group is reacted with a peptide corresponding to a histone amino acid sequence. Transfer of radiolabeled acetate to the peptide is quantified by binding of the peptide to affinity resin (Ait-Si-Ali et al. Nucleic Acids Res. 1998, 26, 3869-3870), phosphocellulose paper (Tanner et al. J. Biol. Chem. 1999, 274, 18157-18160), or scintillation microplates (Wynne Aheme et al. Methods 2002, 26, 245-53) and measurement of the associated radioactivity. In a non-radioactive coupled assay format, the free CoA formed in the acetyltransferase reaction serves as a substrate for α-ketoglutarate dehydrogenase or pyruvate dehydrogenase. Formation of NADH serves as a measure of the rate of acetyltransferase activity (Kim et al. Anal. Biochem. 2000, 280, 308-314).

The most common deacetylase assay methodology involves labeling lysine groups in histone peptides with radiolabeled acetate. The deacetylase enzyme removes the acetyl group as acetate, which is subsequently isolated by extraction and quantified on the basis of its radioactivity (Inoue and Fujimoto, Biochim. Biophys. Acta 1970, 220, 307-316). In a variant of this approach, a scintillation proximity assay, peptides derivatized with radiolabeled acetyl groups are attached to a bead containing scintillant that emits light upon exposure to radiation. In this assay format, cleavage of the acetyl groups causes a decrease in the light emission from the scintillant (Nare, et al., Anal. Biochem. 1999, 267, 390-396). A non-radioactivity-based assay uses peptides containing an acetyllysine group and a fluorescent tag. Reactivity is measured by high-performance liquid chromatography, using the difference in retention time of the acetylated and non-acetylated peptides to isolate and quantify the reaction products (Hoffmann et al. Nucleic Acids Res. 1999, 27, 2057-8; Hoffmann et al. Bioconjug Chem. 2001, 12, 51-5; Hoffmann et al. Arch Pharm (Weinheim) 2001, 334, 248-52). A commercial assay uses a two-step detection protocol. In the first step, a peptide containing an acetyllysine is reacted with a deacetylase for a given period of time. Following this, the reaction is quenched and the exposed lysine is reacted with a developing agent that produces a fluorophore, and the amount of deacetylated lysine is quantified using the fluorescence of the product (Biomol, Plymouth Meeting, Pa., USA). More recently, a two-step, protease-coupled assay was reported, in which a peptide was designed containing a fluorescence resonance energy transfer (FRET) donor-quencher pair and an acetyllysine. After the deacetylase reaction has been allowed to run, the reaction is quenched and the amount of deacetylated peptide is quantified by reaction of the deacetylated peptide with a protease enzyme that cleaves specifically after lysine residues (Frey et al. Presented at 224th National Meeting of the American Chemical Society, Boston, Mass., August 2002; paper MEDI-121, Marcotte et al., Anal. Biochem., 332: 90 (2004)). To date, no continuous, non-radioactive histone deacetylase assays have been reported.

WO0214543 discloses a method of assaying deacetylase/acetylase activity by using a substrate labelled with a fluorophore and at least 1 acetylated lysine residue. The substrate is incubated with the acetylase or deacetylase. The enzyme activity is subsequently be detected which can be performed as fluorescence immunoassay, such as a fluorescence polarization immunoassay.

Wegener, and coworkers (Analytical Biochemistry, 2003, Vol. 321, pages 202-208) describe fluorogenic peptidic substrates specific for HDAC (histone deacetylase) comprising an acetylated lysine moiety and an adjacent 4-methylcoumarin-7-amide moiety at the C-terminus of the peptide chain for use in an HDAC assay. Upon deacetylation of the lysyl moiety, molecules are recognized as substrates for trypsin, which releases highly fluorescent molecules to be measured.

Marcotte and coworkers (Analytical Biochemistry, 2004, Vol. 332, pages 90-99) describe a peptide substrate for deacetylase comprising a fluorophore (6-TAMRA), at least one acetylated lysine residue and QSY-7 as nonfluorescent quenching group. The deacetylated peptide is readily cleaved by trypsin resulting in an enhancement of the 6-TAMRA fluorescence.

Features of the above assay formats limit their utility. Assays based on radioactivity tend to be costly, and require special handling precautions. Also, they are often difficult to perform in a high-throughput manner. Accordingly, improved assays for measuring the activity of acetyltransferases or deacetylases are needed.

## SUMMARY

Provided herein are methods for determining the activity of enzymes that modulate acetylation of a protein substrate, including acetyltransferases and deacetylases. Also provided are methods for identifying compounds that modulate the activity of an acetyltransferase or deacetylase.

In one aspect, the invention provides a method for determining the activity of a deacetylase, comprising: (a) contacting a peptide substrate pool with a deacetylase, wherein the members of said peptide substrate pool comprise a fluorophore and at least one acetylated lysine residue; (b) contacting the peptide substrate pool with a reagent that cleaves members of the peptide substrate pool having non-acetylated lysine residues; and (c) determining the fluorescence polarization value of the peptide substrate pool, wherein a decrease in the fluorescence polarization value of the peptide substrate pool is indicative of deacetylase activity.

In certain embodiments, the members of said peptide substrate pool may further comprise a bulky group and at least one acetylated lysine residue located between the fluorophore and the bulky group. The bulky group may be, for example, biotin, a biotin-avidin complex, or a biotin-streptavidin complex.

In certain embodiments, the members of said peptide substrate pool may further comprise a binding site and said at least one acetylated lysine residue is located between the fluorophore and the binding site. In such embodiments, the method for determining the activity of a deacetylase may further comprise contacting the peptide substrate pool with a binding moiety that binds to said binding site. In various embodiments, the binding site/binding moiety pairs may be, for example, an antigen/antibody, biotin/avidin, biotin/streptavidin, and an Fc region or a portion thereof/protein A or protein G.

In certain embodiments, the deacetylase may be, for example, a histone deacetylase (HDAC) or a sirtuin. In certain embodiments, the sirtuin may be, for example, a SIRT1 protein.

In certain embodiments, the reagent that cleaves the peptide substrate may be a protease, such as, for example, lysylendopeptidase, endoproteinase, Lys-C, plasmin, calpain, or trypsin.

In certain embodiments, the fluorophore may be MR121 or 5TMR.

In certain embodiments, the sequence of the peptide substrate may be derived from a histone, an HMG protein, p53, c-Myb, GATA-1, EKLF, MyoD, E2F, dTCF, or HIV Tat, or a fragment thereof.

In another aspect, the invention provides, a method for identifying a compound that modulates a deacetylase, comprising: (a) contacting a peptide substrate pool with a deacetylase in the presence of a test compound, wherein the members of said peptide substrate pool comprise a fluorophore and at least one acetylated lysine residue; (b) contacting the peptide substrate pool with a reagent that cleaves members of the peptide substrate pool having non-acetylated lysine residues; and (c) determining the fluorescence polarization value of the peptide substrate pool, wherein a change in the fluorescence polarization value of the peptide substrate pool in the presence of the test compound as compared to a control is indicative of a compound that modulates the activity of the deacetylase.

In certain embodiments, the members of said peptide substrate pool may further comprise a bulky group and at least one acetylated lysine residue located between the fluorophore and the bulky group. The bulky group may be, for example, biotin, a biotin-avidin complex, or a biotin-streptavidin complex.

In certain embodiments, the members of said peptide substrate pool may further comprise a binding site and said at least one acetylated lysine residue is located between the fluorophore and the binding site. In such embodiments, the method for identifying a compound that modulates a deacetylase may further comprise contacting the peptide substrate pool with a binding moiety that binds to said binding site. In various embodiments, the binding site/binding moiety pairs may be, for example, an antigen/antibody, biotin/avidin, biotin/streptavidin, and an Fc region or a portion thereof/protein A or protein G.

In certain embodiments, the deacetylase may be, for example, a histone deacetylase (HDAC) or a sirtuin. In certain embodiments, the sirtuin may be, for example, a SIRT1 protein.

In certain embodiments, the reagent that cleaves the peptide substrate may be a protease, such as, for example, lysylendopeptidase, endoproteinase, Lys-C, plasmin, calpain, or trypsin.

In certain embodiments, the fluorophore may be MR121 or 5TMR.

In certain embodiments, the sequence of the peptide substrate may be derived from a histone, an HMG protein, p53, c-Myb, GATA-1, EKLF, MyoD, E2F, dTCF, or HIV Tat, or a fragment thereof.

In certain embodiments, a compound that increases the activity of the deacetylase is identified. In such embodiments, a decrease in the fluorescence polarization value of the peptide substrate pool in the presence of the test compound as compared to a control is indicative of a compound that increases the activity of the deacetylase.

In certain embodiments, a compound that inhibits the activity of the deacetylase is identified. In such embodiments, an increase in the fluorescence polarization value of the peptide substrate pool in the presence of the test compound as compared to a control is indicative of a compound that inhibits the activity of the deacetylase.

In certain embodiments, the compound is a small molecule.

In another aspect, the invention provides a method for determining the activity of an acetyltransferase, comprising: (a) contacting a peptide substrate pool with an acetyltransferase, wherein the members of said peptide substrate pool comprise a fluorophore and at least one lysine residue; (b) contacting the peptide substrate pool with a reagent that cleaves members of the peptide substrate pool having non-acetylated lysine residues; and (c) determining the fluorescence polarization value of the peptide substrate pool, wherein an increase in the fluorescence polarization value of the peptide substrate pool is indicative of acetyltransferase activity.

In certain embodiments, the members of said peptide substrate pool may further comprise a bulky group and at least one lysine residue located between the fluorophore and the bulky group. The bulky group may be for example, biotin, a biotin-avidin complex, or a biotin-streptavidin complex.

In certain embodiments, the members of said peptide substrate pool may further comprise a binding site and at least one lysine residue located between the fluorophore and the binding site. In such embodiments, the method for determining the activity of an acetyltransferase may further comprises contacting the peptide substrate pool with a binding moiety that binds to said binding site. In various embodiments, the binding site/binding moiety pairs may be, for example, an antigen/antibody, biotin/avidin, biotin/streptavidin, and an Fc region or a portion thereof/protein A or protein G.

In certain embodiments, the acetyltransferase may be, for example, Gcn5 or p300/CBP.

In certain embodiments, the reagent that cleaves the peptide substrate may be a protease, such as, for example, lysylendopeptidase, endoproteinase, Lys-C, plasmin, calpain, or trypsin.

In certain embodiments, the fluorophore may be, for example, MR121 or 5TMR.

In certain embodiments, the sequence of the peptide substrate may be derived from a histone, an HMG protein, p53, c-Myb, GATA-1, EKLF, MyoD, E2F, dTCF, or HIV Tat, or a fragment thereof.

In another aspect, the invention provides a method for identifying a compound that modulates an acetyltransferase, comprising: (a) contacting a peptide substrate pool with an acetyltransferase in the presence of a test compound, wherein the members of said peptide substrate pool comprise a fluorophore and at least one lysine residue located between the fluorophore and the binding site; (b) contacting the peptide substrate pool with a reagent that cleaves molecules of the peptide substrate having non-acetylated lysine residues; and (c) determining the fluorescence polarization value of the peptide substrate pool, wherein a change in the fluorescence polarization value of the peptide substrate pool in the presence of the test compound as compared to a control is indicative of a compound that modulates the activity of the acetyltransferase.

In certain embodiments, the members of said peptide substrate pool may further comprise a bulky group and at least one lysine residue located between the fluorophore and the bulky group. The bulky group may be for example, biotin, a biotin-avidin complex, or a biotin-streptavidin complex.

In certain embodiments, the members of said peptide substrate pool may further comprise a binding site and at least one lysine residue located between the fluorophore and the binding site. In such embodiments, the method for identifying a compound that modulates an acetyltransferase may further comprises contacting the peptide substrate pool with a binding moiety that binds to said binding site. In various embodiments, the binding site/binding moiety pairs may be, for example, an antigen/antibody, biotin/avidin, biotin/streptavidin, and an Fc region or a portion thereof/protein A or protein G.

In certain embodiments, the acetyltransferase may be, for example, Gcn5 or p300/CBP.

In certain embodiments, the reagent that cleaves the peptide substrate may be a protease, such as, for example, lysylendopeptidase, endoproteinase, Lys-C, plasmin, calpain, or trypsin.

In certain embodiments, the fluorophore may be, for example, MR121 or 5TMR.

In certain embodiments, the sequence of the peptide substrate may be derived from a histone, an HMG protein, p53, c-Myb, GATA-1, EKLF, MyoD, E2F, dTCF, or HIV Tat, or a fragment thereof.

In certain embodiments, a compound that inhibits the activity of the acetyltransferase is identified. In such embodiments, a decrease in the fluorescence polarization value of the peptide substrate pool upon contact with the acetyltransferase in the presence of the test compound as compared to a control is indicative of a compound that inhibits

the activity of the acetyltransferase.

In certain embodiments, a compound that increases the activity of the acetyltransferase is identified. In such embodiments, an increase in the fluorescence polarization value of the peptide substrate pool upon contact with the acetyltransferase in the presence of the test compound as compared to a control is indicative of a compound that increases the activity of the acetyltransferase.

In certain embodiments, the compound is a small molecule.

In another aspect, the invention provides a method for determining the activity of an enzyme that modulates acetylation of a peptide substrate, comprising: (a) contacting a peptide substrate pool with an enzyme that modulates acetylation; (b) contacting the peptide substrate pool with a cleavage reagent; and (c) determining the fluorescence polarization value of the peptide substrate pool, wherein a change in the fluorescence polarization value of the peptide substrate pool is indicative of activity of the enzyme that modulates acetylation.

In another aspect, the invention provides a method for identifying a compound that modulates the activity of an enzyme that modulates acetylation of a peptide substrate, comprising: (a) contacting a peptide substrate pool with an enzyme that modulates acetylation in the presence of a test compound; (b) contacting the peptide substrate pool with a cleavage reagent; and (c) determining the fluorescence polarization value of the peptide substrate pool, wherein a change in the fluorescence polarization value of the peptide substrate pool in the presence of the test compound is indicative of a compound that modulates the activity of the enzyme that modulates acetylation.

The practice of the present methods will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. L Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

## DETAILED DESCRIPTION

### 1. Definitions

As used herein, the following terms and phrases shall have the meanings set forth below. Unless defined otherwise, all technical and scientific terms used herein have the same meaning as commonly understood to one of ordinary skill in the art.

The singular forms "a," "an," and "the" include plural reference unless the context clearly dictates otherwise.

The terms "comprise" and "comprising" are used in the inclusive, open sense, meaning that additional elements may be included.

The term "conserved residue" refers to an amino acid that is a member of a group of amino acids having certain common properties. The term "conservative amino acid substitution" refers to the substitution (conceptually or otherwise) of an amino acid from one such group with a different amino acid from the same group. A functional way to define common properties between individual amino acids is to analyze the normalized frequencies of amino acid changes between corresponding proteins of homologous organisms (Schulz, G. E. and R. H. Schirmer., Principles of Protein Structure, Springer-Verlag). According to such analyses, groups of amino acids may be defined where amino acids within a group exchange preferentially with each other, and therefore resemble each other most in their impact on the overall protein structure (Schulz, G. E. and R H. Schirmer, Principles of Protein Structure, Springer-Verlag). One example of a set of amino acid groups defined in this manner include: (i) a charged group, consisting of Glu and Asp, Lys, Arg and His, (ii) a positively-charged group, consisting of Lys, Arg and His, (iii) a negatively-charged group, consisting of Glu and Asp, (iv) an aromatic group, consisting of Phe, Tyr and Trp, (v) a nitrogen ring group, consisting of His and Trp, (vi) a large aliphatic nonpolar group, consisting of Val, Leu and Ile, (vii) a slightly-polar group, consisting of Met and Cys, (viii) a small-residue group, consisting of Ser, Thr, Asp, Asn, Gly, Ala, Glu, Gln and Pro, (ix) an aliphatic group consisting of Val, Leu, Ile, Met and Cys, and (x) a small hydroxyl group consisting of Ser and Thr.

The term "including" is used to mean "including but not limited to". "Including" and "including but not limited to" are used interchangeably.

The term "mammal" is known in the art, and exemplary mammals include humans, primates, livestock animals

(including bovines, porcines, etc.), companion animals (e.g., canines, felines, etc.) and rodents (e.g., mice and rats).

The term "modulate", when used in reference to the activity of an acetyltransferase or deacetylase, refers to the up regulation (e.g., activation or stimulation), down regulation (e.g., inhibition or suppression), or other change in a quality of such acetyltransferase or deacetylase activity.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, MD. In one embodiment, the percent identity of two sequences can be determined by the GCG program with a gap weight of 1, e.g., each amino acid gap is weighted as if it were a single amino acid or nucleotide mismatch between the two sequences.

Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "substantially homologous" when used in connection with amino acid sequences, refers to sequences which are substantially identical to or similar in sequence with each other, giving rise to a homology of conformation and thus to retention, to a useful degree, of one or more biological (including immunological) activities. The term is not intended to imply a common evolution of the sequences.

The term "synthetic" is art-recognized and refers to production by *in vitro* chemical or enzymatic synthesis.

The term "therapeutic agent" is art-recognized and refers to any chemical moiety that is a biologically, physiologically, or pharmacologically active substance that acts locally or systemically in a subject. The term also means any substance intended for use in the diagnosis, cure, mitigation, treatment or prevention of disease or in the enhancement of desirable physical or mental development and/or conditions in an animal or human.

The term "therapeutic effect" is art-recognized and refers to a local or systemic effect in animals, particularly mammals, and more particularly humans caused by a pharmacologically active substance. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art.

## 2. Acetyltransferase/Deacetylase Assays

Provided herein are methods for determining the activity of acetyltransferase (acetylase) and deacetylase enzymes. The methods may involve, for example, contacting a substrate peptide pool with an acetyltransferase or deacetylase enzyme, cleaving the substrate peptide pool, and determining the fluorescence polarization value of the substrate pool. In other embodiments, the invention provides methods for identifying compounds that modulate the activity an acetyltransferase or deacetylase enzyme. The methods may involve, for example, contacting a substrate peptide pool with an acetyltransferase or deacetylase enzyme in the presence of a test compound, cleaving the substrate peptide pool, and determining the fluorescence polarization value of the substrate pool.

The activity of an acetyltransferase (or acetylase) enzyme are determined using the methods described herein. An acetylase is an enzyme that catalyzes a reaction by which an acetyl group ($CH_3CO$-) is transferred from a certain substance (for example, acetyl-CoA) to a peptide. Exemplary acetylases include, for example, members of the GNAT (Gcn5-realted N-acetyltransferase) superfamily, such as, for example, Hat1, Gcn5, PCAF, Elp3, and Hpa2; members of the MYST (MOZ, Ybf2,/Sas3, Sas2, and Tip60) family, such as, for example, Sas2, Sas3, Esa1, MOF, Tip60, MOZ, MORF, and HBO1; p300/CBP; nuclear receptor coactivators, such as, for example, SRC-1, ACTR, and TIF2; $TAF_{II}250$; TFIIIC proteins, such as, for example, TFIIIC220, TFillCII0 and TFIIIC90. Homologs, e.g., orthologs and paralogs, domains, fragments, variants and derivatives of the foregoing may also be used in accordance with the methods described herein. Acetylases and their substrates are reviewed, for example, in Sterner and Berger, Microbiol. Mol. Biol. Rev., 64: 453-459 (2000).

The activity of a deacetylase enzyme are determined using the methods described herein. A deacetylase is an enzyme that releases an acetyl group from an acetylated peptide. Exemplary deacetylase enzymes include, for example, histone deacetylases (HDACs) class I or II and HDACs class III (or sirtuins). Class I HDACs (HDACs 1, 2, 3 and 8) bear similarity to the yeast RPD3 protein, are located in the nucleus and are found in complexes associated with transcriptional corepressors. Class II HDACs (HDACs 4, 5, 6, 7 and 9) are similar to the yeast HDA1 protein, and have both nuclear and cytoplasmic subcellular localization. Both Class I and II HDACs are inhibited by hydroxamic acid-based HDAC inhibitors, such as SAHA. Class III HDACs form a structurally distant class of NAD dependent enzymes that are related to the yeast SIR2 proteins and are not inhibited by hydroxamic acid-based HDAC inhibitors.

Exemplary HDAC class I or II enzymes that may be used in accordance with the methods described herein include, for example, human HDACs 1-8, e.g., HDAC-1 (GenBank Accession No. AAC50475 (nucleotide), U50079 (amino acid)), HDAC-2 (GenBank Accession No. AAC50814 (nucleotide), U31814 (amino acid)), HDAC-3 (GenBank Accession No. AAB88241 (nucleotide), U75697 (amino acid)), HDAC-4 (GenBank Accession No. BAA22957 (nucleotide), AB006626 (amino acid)), HDAC-5 (GenBank Accession No. BAA25526 (nucleotide), AB011172 (amino acid)), HDAC-6 (GenBank Accession No. AAD29048 (nucleotide), AJ011972 (amino acid)), HDAC-7 (GenBank Accession No. AAF63491.1 (nucleotide), AF239243 (amino acid)), or HDAC-8 (GenBank Accession No. AAF73076.1 (nucleotide), AF230097 (amino acid)), as well as homologs, e.g., orthologs and paralogs, domains, fragments, variants and derivatives of the foregoing.

In other embodiments, a deacetylase that may be used in accordance with the methods described herein is a sirtuin protein. A sirtuin protein refers to a member of the sirtuin deacetylase protein family, or preferably to the sir2 family, which include yeast Sir2 (GenBank Accession No. P53685), *C. elegans* Sir-2.1 (GenBank Accession No. Nip_501912), and human SIRT1 (GenBank Accession No. NM_012238 and NP_036370 (or AF083106)) and SIRT2 (GenBank Accession No. NM_012237, NM_030593, NP_036369, NP_085096, and AF083107) proteins. Other family members include the four additional yeast Sir2-like genes termed "*HST* genes" (homologues of Sir two) HST1, HST2, HST3 and HST4, and the five other human homologues hSIRT3, hSIRT4, hSIRT5, hSIRT6 and hSIRT7 (Brachmann et al. (1995) Genes Dev. 9:2888 and Frye et al. (1999) BBRC 260:273). Homologs, e.g., orthologs and paralogs, domains, fragments, variants and derivatives of the foregoing may also be used in accordance with the methods described herein.

In an exemplary embodiment, the methods described herein may be used to determine the activity of a SIRT1 protein. A SIRT1 protein refers to a member of the sir2 family of sirtuin deacetylases. In one embodiment, a SIRT1 protein includes yeast Sir2 (GenBank Accession No. P53685), C. elegans Sir-2.1 (GenBank Accession No. NP_501912), human SIRT1 (GenBank Accession No. NM_012238 or NP_036370 (or AF083106)), and human SIRT2 (GenBank Accession No. NM_012237, NM_030593, NP_036369, NP_085096, or AF083107) proteins, and equivalents and fragments thereof. In another embodiment, a SIRT1 protein includes a polypeptide comprising a sequence consisting of, or consisting essentially of, the amino acid sequence set forth in GenBank Accession Nos. NP_036370, NP_501912, NP_085096, NP_036369, or P53685. SIRT1 proteins include polypeptides comprising all or a portion of the amino acid sequence set forth in GenBank Accession Nos. NP_036370, NP_501912, NP_085096, NP_036369, or P53685; the amino acid sequence set forth in GenBank Accession Nos. NP_036370, NP_501912, NP_085096, NP_036369, or P53685 with 1 to about 2, 3, 5, 7, 10, 15, 20, 30, 50, 75 or more conservative amino acid substitutions; an amino acid sequence that is at least 60%, 70%, 80%, 90%, 95%, 96%, 97%, 98%, or 99% identical to GenBank Accession Nos. NP_036370, NP_501912, NP_085096, NP_036369, or P53685, and functional fragments thereof. SIRT1 proteins also

include homologs (e.g., orthologs and paralogs), variants, or fragments, of GenBank Accession Nos. NP_036370, NP_501912, NP_085096, NP_036369, or P53685.

In one embodiment, the methods described herein may be used to determine the activity of a SIRT3 protein. A SIRT3 protein refers to a member of the sirtuin deacetylase protein family and/or to a homolog of a SIRT1 protein. In one embodiment, a SIRT3 protein includes human SIRT3 (GenBank Accession No. AAH01042, NP_036371, or NP_001017524) and mouse SIRT3 (GenBank Accession No. NP_071878) proteins, and equivalents and fragments thereof. In another embodiment, a SIRT3 protein includes a polypeptide comprising a sequence consisting of, or consisting essentially of, the amino acid sequence set forth in GenBank Accession Nos. AAH01042, NP_036371, NP_001017524, or NP_071878. SIRT3 proteins include polypeptides comprising all or a portion of the amino acid sequence set forth in GenBank Accession AAH01042, NP_036371, NP_001017524, or NP 071878; the amino acid sequence set forth in GenBank Accession Nos. AAH01042, NP_036371, NP_001017524, or NP_071878 with 1 to about 2, 3, 5, 7, 10, 15, 20, 30, 50, 75 or more conservative amino acid substitutions; an amino acid sequence that is at least 60%, 70%, 80%, 90%, 95%, 96%, 97%, 98%, or 99% identical to GenBank Accession Nos. AAH01042, NP_036371, NP_001017524, or NP_071878, and functional fragments thereof. SIRT3 proteins also include homologs (e.g., orthologs and paralogs), variants, or fragments, of GenBank Accession Nos. AAH01042, NP_036371, NP_001017524, or NP_071878.

In another embodiment, a biologically active portion of a sirtuin may be used in accordance with the methods described herein. A biologically active portion of a sirtuin refers to a portion of a sirtuin protein having a biological activity, such as the ability to deacetylate. Biologically active portions of sirtuins may comprise the core domain of a sirtuin. Biologically active portions of SIRT1 having GenBank Accession No. NP_036370 that encompass the NAD+ binding domain and the substrate binding domain, for example, may include without limitation, amino acids 62-293 of GenBank Accession No. NP_036370, which are encoded by nucleotides 237 to 932 of GenBank Accession No. NM_012238. Therefore, this region is sometimes referred to as the core domain. Other biologically active portions of SIRT1, also sometimes referred to as core domains, include about amino acids 261 to 447 of GenBank Accession No. NP_036370, which are encoded by nucleotides 834 to 1394 of GenBank Accession No. NM_012238; about amino acids 242 to 493 of GenBank Accession No. NP_036370, which are encoded by nucleotides 777 to 1532 of GenBank Accession No. NM_012238; or about amino acids 254 to 495 of GenBank Accession No. NP_036370, which are encoded by nucleotides 813 to 1538 of GenBank Accession No. NM_012238. In another embodiment, a biologically active portion of a sirtuin may be a fragment of a SIRT3 protein that is produced by cleavage with a mitochondrial matrix processing peptidase (MPP) and/or a mitochondrial intermediate peptidase (MIP).

A wide variety of peptide substrates may be used in accordance with the methods described herein. When determining the activity of an acetyltransferase, the peptide substrate utilized in the reaction comprises at least one non-acetylated lysine residue. When determining the activity of a deacetylase, the peptide substrate utilized in the reaction comprises at least one acetylated lysine residue.

The sequence of the peptide substrate may be obtained, or derived, from a protein that may be acetylated or deacetylated by an acetyltransferase or deacetylase, respectively. Exemplary substrates for acetyltransferases and deacetylases include, for example, histones (e.g., H1, H2, H2A, H2B, H3 and H4), nonhistone chromatin proteins (e.g., HMG1, HMG2, Yeast Sin1, HMG14, HMG17, and HMG I(Y)), transcriptional activators (e.g., p53, c-Myb, GATA-1, EKLF, MyoD, E2F, dTCF, and HIV Tat), nuclear receptor coactivators (e.g., ACTR, SRC-1, TIF2), general transcription factors (e.g., TFIIE and TFIIF), importin-$\alpha$7, Rch1, and $\alpha$-tubulin. Substrate peptides used in accordance with the methods described herein may comprise an entire substrate protein or a portion thereof containing at least one lysine residue. In certain embodiments, it may be desirable to modify the sequence of a substrate protein, or a fragment thereof, to remove one or more lysine and/or arginine residues. For example, it may be desirable to have a substrate peptide that comprises lysine residues but no other positively charged residues (such as arginine) such that upon cleavage with a protease, such as trypsin, cleavage of the substrate peptide will be dependent upon the acetylation state of the lysine residues in the substrate and independent of arginine residues. Additionally, it may be desirable to have a substrate peptide that contains one or more lysine residues located only in desired locations within the substrate peptide, e.g., between a fluorophore a high molecular weight or bulky group. In certain embodiments, it may be desirable to have a substrate peptide that contains only a single lysine residue and is free of any arginine residues. In an exemplary embodiment, a lysine residue may be located in the substrate peptide such that upon cleavage of the peptide substrate at or near the site of the lysine residue, the size differential between an uncleaved peptide substrate and the portion of a cleaved peptide substrate containing the fluorophore is sufficient to provide a change in fluorescence polarization. One or more lysine and/or arginine residues may be removed from a peptide substrate sequence by replacing the amino acid residue with a different amino acid residue or by deleting the amino acid residue from the sequence without substitution of a different amino acid. In certain embodiments, one or more lysine and/or arginine residues may be replaced using a conservative amino acid substitution wherein said substitution is not lysine or arginine.

Substrate peptides that may be used in accordance with the methods described herein can be synthesized according to conventional methods. The substrate peptides may include naturally occurring peptides, peptides prepared by genetic recombination techniques, and synthetic peptides. The peptides may be fused with other peptides (for example,

glutathione-S-transferase, HA tag, FLAG tag, etc.) for convenience of purification, etc. Further, the peptide may comprise structural units other than amino acids so long as it serves as a substrate for a deacetylase, or acetyltransferase, and a protease. Typically, the synthesis of a peptide is achieved by adding amino acids, residue by residue, from the carboxyl terminus of the amino acid sequence of interest. Further, some of the peptide fragments synthesized in that way may be linked together to from a larger peptide molecule. For measuring deacetylase activity, the substrate peptide needs to be acetylated before the reaction is conducted. An exemplary method of amino acid acetylation includes acetylation of amino acids, whose α-amino groups and side-chain amino groups are blocked with protecting groups, with acetic anhydride, N-hydroxysuccinimide acetate, or similar reagents. These acetylated amino acids are then used to synthesize peptides comprising acetylated lysine residues, for example, using the solid-phase method. Generally, acetylated peptides can be synthesized using a peptide synthesizer according to the Fmoc method. For example, commercial suppliers, who provide custom peptide synthesis services, can synthesize peptides having specified amino acid sequences comprising residues acetylated at predetermined positions.

The substrate peptide also comprises at least one fluorophore. Exemplary fluorophores that may be used in accordance with the methods described herein are provided below in Table 1. Methods for labeling a peptide with a fluorophore are known in the art, and thus, can be conducted according to conventional methods. The fluorophore may be covalently linked or conjugated to the peptide so as not to interfere with emission of fluorescence from the label, acetylation or deacetylation of the lysine residue(s), or cleavage of the substrate peptide with a protease.

Table 1. Commercially available fluorophores and their excitation and emission maxima.

| **Dye** | **Excitation/Emission (nm)** |
|---|---|
| AF 350 | 346/442 |
| AF 430 | 433/539 |
| AF 488 | 495/519 |
| AF 532 | 532/554 |
| AF 546 | 556/573 |
| AF 568 | 578/603 |
| AF 594 | 590/617 |
| AF 633 | 632/647 |
| AF 647 | 650/668 |
| AF 660 | 663/690 |
| AF 680 | 679/702 |
| Dintrophenyl | 349/NA |
| AMCA | 346/442 |
| Cascade Blue | 400/420 |
| Marina Blue | 365/460 |
| Fluorescein/FITC | 494/518 |
| Oregon Green 488 | 496/524 |
| Rhodamine Green | 505/527 |
| BODIPY FL | 504/513 |
| BODIPY TMR | 535/574 |
| BODIPY TR | 588/617 |
| Oregon Green 514 | 511/530 |
| Rhodamine Red | 570/590 |
| Tetramethylrhodamine | 555/580 |
| Texas Red | 595/615 |

(continued)

| Dye | Excitation/Emission (nm) |
|---|---|
| BODIIPY 630/650 | 632/640 |
| BODTPY 650/665 | 651/660 |
| QSY7 | 560/NA |
| FluorX | 494/520 |
| Cy2 bis | 489/506 |
| Cy3 mono | 550/570 |
| Cy3.5 mono | 581/596 |
| Cy5 mono | 649/670 |
| Cy5.5 mono | 675/694 |
| Cy7 mono | 743/767 |
| DEAC | 432/472 |
| R6G | 524/552 |
| TAMRA | 547/573 |
| MR121 | 635/680 |

In certain embodiments, the substrate peptide further comprises a high molecular weight group or a bulky group. The high molecular weight or bulky group is separated from the fluorophore by at least one lysine residue. When the lysine residue is in the non-acetylated state, the substrate peptide is susceptible to cleavage at or near the lysine residue by a cleavage reagent, such as a protease. When the lysine residue is in the acetylated state, the substrate peptide is resistant to cleavage and remains intact upon contact with a cleavage reagent. Upon cleavage, the fluorophore is separated from the high molecular weight or bulky group thereby decreasing the fluorescent polarization value of the sample.

Fluorescence polarization (FP) is based upon the theory that when a fluorescently labeled molecule is excited with plane-polarized light of the correct wavelength it will emit polarized light after its characteristic emission lifetime (usually nanoseconds). During the time between excitation and emission (fluorescence lifetime), the molecule tumbles randomly with respect to the original plane of excitation. The rate of tumbling is directly proportional to its molecular volume or size. Small molecules tumble rapidly, while large molecules tumble much more slowly. If the molecule tumbles rapidly during its fluorescence lifetime, the fluorescence emission is depolarized relative to the excitation. If the fluorophore is associated with a much bigger molecule, the tumbling is slow and the observed emission remains more polarized relative to the excitation. Thus by measuring the extent of fluorescence polarization, one can tell if a fluorophore is associated with a larger molecule.

One advantage of FP is that it can be utilized in homogeneous assays and therefore is useful for high throughput screening (HTS) assays. FP is also amenable to performing assays in real-time, directly in solution and without the need for an immobilized phase. Polarization values can be measured repeatedly and after the addition of reagents since measuring the samples is rapid and does not destroy the sample.

The polarization value (P) for a given molecule is proportional to the molecule's rotational relaxation time, or the amount of time it takes the molecule to rotate through an angle of 68.5˚. The smaller the rotational correlation time, the faster the molecule rotates, and the less polarization will be observed. The larger the rotational correlation time, the slower the molecule rotates, and the more polarization will be observed. Rotational relaxation time is related to viscosity (i), absolute temperature (T), molecular volume (V), and the gas constant (R). The rotational relaxation time is generally calculated according to the following formula:

$$\text{Rotational Relaxation Time} = 3\eta V/RT$$

As can be seen from the above equation, if temperature and viscosity are maintained constant, then the rotational relaxation time, and therefore, the polarization value, is directly related to the molecular volume. Accordingly, the larger the molecule, the higher its fluorescent polarization value, and conversely, the smaller the molecule, the smaller its

fluorescent polarization value.

In the performance of the methods described herein, the starting peptide substrate has a slow rotational correlation time. The fluorophore is attached to a portion of the peptide substrate that, upon cleavage at the lysine residue, is released and exhibits a relatively fast rotational correlation time (e.g., in comparison to the rotational correlation time of the uncleaved peptide substrate). Cleavage of the peptide substrate is indirectly proportional to the acetylation state of the peptide substrate and directly proportional to the fluorescence polarization of the peptide substrate. Therefore, upon an increase in acetylation of the peptide substrate (for example, upon exposure to an acetyltransferase), cleavage of the peptide substrate is decreased and the fluorescence polarization value of the peptide substrate will increase (e.g., the amount of rotation of the fluorophore remains low because it is associated with the larger substrate peptide molecule therefore increasing the rotational correlation time). Conversely, upon a decrease in the acetylation of the peptide substrate (for example, upon exposure to a deacetylase), the cleavage of the peptide substrate is increased and the fluorescence polarization value of the peptide substrate will decrease (e.g., the amount of rotation of the fluorophore increases upon release from the larger substrate peptide molecule therefore decreasing the rotational correlation time).

Generally, the fluorescence polarization level is calculated using the following formula:

$$P=[I(\parallel)-I(\perp)]/[I(\parallel)+I(\perp)]$$

Where I(||) is the fluorescence detected in the plane parallel to the excitation light, and I(⊥) is the fluorescence detected in the plane perpendicular to the excitation light.

In performing screening assays, e.g., for potential inhibitors, enhancers, agonists or antagonists of acetyltransferases or deacetylases, the change in fluorescence polarization of the substrate peptide is compared in the presence and absence of different compounds, to determine whether these different compounds have any effect on the enzymes being examined. In particular, in the presence of an inhibitor of an acetyltransferase, the fluorescence polarization as compared to a control will decrease, as a greater proportion of the peptide substrate pool will be non-acetylated and therefore can be cleaved at the non-acetylated lysine residue to release the fluorophore from the larger peptide substrate molecule. In the presence of an activator of an acetyltransferase, the fluorescence polarization as compared to a control will increase, as a greater proportion of the peptide substrate pool will be acetylated and therefore will not be cleaved so that the fluorophore remains associated with the larger peptide substrate molecule. In the presence of an inhibitor of a deacetylase, the fluorescence polarization as compared to a control will increase, as a greater proportion of the peptide substrate pool will remain acetylated and therefore will not be cleaved so that the fluorophore remains associated with the larger peptide substrate molecule. Finally, in the presence of an activator of a deacetylase, the fluorescence polarization as compared to a control will decrease, as a greater proportion of the peptide substrate pool will become non-acetylated and therefore can be cleaved at the non-acetylated lysine residue to release the fluorophore from the larger peptide substrate molecule.

Fluorescence polarization may be monitored in any of three different states: steady state, transient state, or dynamic state. In transient state FP, the excitation light source is flashed on the sample and polarization of the emitted light is monitored by turning on the photomultiplier tube after the excitation light source is turned off. This reduces interference from light scatter, as fluorescence lasts longer than light scatter, but some fluorescence intensity is lost. In steady state FP, excitation light and emission monitoring are continuous (i.e., the excitation source and photomultiplier tube are on continuously). This results in measurement of an average tumbling time over the monitoring period and includes the effects of scattered light. Dynamic FP may be monitored in either the time- or frequency-domain. Dynamic fluorescence techniques involve determining the lifetime of the fluorescent molecule in nanoseconds. The theory of dynamic fluorescence monitoring is described in "Principles of Fluorescence Spectroscopy" (Lakowicz, Plenum Press, 1983). Whereas steady state FP provides an average or "snapshot" of the fluorescence phenomena, dynamic FP allows one to observe the individual contributions of the fluorescent components in the system being studied. Use of these three fluorescence techniques is described by Kumke, et al. (1995. Anal. Chem. 67, 3945-3951), and Devlin, et al. (1993. Clin. Chem. 39, 1939-1943). Methodology for carrying out fluorescence polarization is set forth in "Fluorescence Polarization. Technical Resource Guide, Third Edition", available from PanVera Corporation, Madison, Wis., USA.

High molecular weight groups or bulky groups that may be used in association with the peptide substrate described herein include anything that is sufficient to permit a differential in the fluorescence polarization value of the fluorophore when it is associated with the uncleaved peptide substrate as opposed to when it has been cleaved, or released, from the peptide substrate molecule. Examples of high molecular weight groups or bulky groups include, for example, polypeptides, nucleic acids, carbohydrates, etc. In certain embodiments, the high molecular weight or bulky group may simply be a portion of the protein substrate itself (e.g., the lysine residue is placed such that a very small portion of a larger substrate molecule is released upon cleavage). In certain embodiments, the high molecular weight or bulky group may be covalently attached or non-covalently bound to the peptide substrate. In certain embodiments,

the substrate peptide may comprise a binding site and the high molecular weight group or bulky group may be a binding moiety that is non-covalently bound to the binding site. Association between the binding site and the binding moiety may be carried out either before or after cleavage of the peptide substrate with a cleavage reagent. Various binding site/binding moiety pairs may be used in association with the peptide substrate molecules, such as for example, a biotin/avidin complex , a biotin/streptavidin complex, an Fc region (or portion thereof)/protein A or protein G, an antigen (e.g., a peptide sequence)/antibody, a ligand/receptor molecule, an aptamer/aptamer binding pair, etc. When using an antigen as the binding site, the antigen may be a portion of the substrate peptide itself, or may be a sequence that is added on, such as, glutathione S-transferase (GST), or an HA, myc, or FLAG tag, for the purpose of interaction with a given antibody. Methods for producing antibodies are well known in the art. Antibodies specific for a variety of sequences, such as, for example, GST, HA, Myc or FLAG tags, are commercially available.

Exemplary peptide substrates for Sirt1, Sirt2 and Sirt3 deacetylases that may be used in accordance with the methods described herein are shown below in Table 2.

Table 2. Sirt1, Sirt2 and Sirt3 peptide substrates.

| Enzyme/ Basis for Peptide sequence | Sequence | SEQ ID NO |
|---|---|---|
| Sirt1/ p53 | Ac-EE-K(biotin)-GQSTSSHS-K(Ac)-Nle-STEG-K (MR121)-EE-$NH_2$ | SEQ ID NO: 1 |
| Sirt1/ p53 | Ac-EE-K(biotin)-GQSTSSHS-K(Ac)-Nle-STEG-K(5-TMR)-EE-$NH_2$ | SEQ ID NO: 2 |
| Sirt1/ PGC1alpha | Biotin-TNPAIV-K(Ac)-TENS-K(MR121)-$NH_2$ | SEQ ID NO: 3 |
| Sirtl/ PGClalpha | Biotin-TNPAIV-K(Ac)-TENS-K(5-TMR)-$NH_2$ | SEQ ID NO: 4 |
| Sirt1/ PGC1alpha | Biotin-QHLQA-K(Ac)-PTTLS-K(MR121)-$NH_2$ | SEQ ID NO: 5 |
| Sirt1/ PGClalpha | Biotin-QHLQA-K(Ac)-PTTLS-K(5-TMR)-$NH_2$ | SEQ ID NO: 6 |
| Sirt2/ alpha-Tubulin | Biotin-MPSD-K(Ac)-TIGG-K(MR121)-$NH_2$ | SEQ ID NO: 7 |
| Sirt2/ alpha-Tubulin | Biotin-MPSD-K(Ac)-TIGG-K(5-TMR)-$NH_2$ | SEQ ID NO: 8 |
| Sirt2/ alpha-Tubulin | Biotin-Nle-PSD-K(Ac)-TIGG-K(MR121)-$NH_2$ | SEQ ID NO: 9 |
| Sirt2/ alpha-Tubulin | Biotin-Nle-PSD-K(Ac)-TIGG-K(5-TMR)-$NH_2$ | SEQ ID NO: 10 |
| Sirt2/ alpha-Tubulin | Biotin-GQ-Nle-PSD-K(Ac)-TIGG-K(MR121)-$NH_2$ | SEQ ID NO: 11 |
| Sirt2/ alpha-Tubulin | Biotin-GQ-Nle-PSD-K(Ac)-TIGG-K(5-TMR)-$NH_2$ | SEQ ID NO: 12 |
| Sirt3/ Acetyl CoA Synthase 2 | Biotin-SG-K(Ac)-IM-K(MR121)-$NH_2$ | SEQ ID NO: 13 |
| Sirt3/ Acetyl CoA Synthase 2 | Biotin-SG-K(Ac)-IM-K(5-TMR)-$NH_2$ | SEQ ID NO: 14 |
| Sirt3/ Acetyl CoA Synthase 2 | Biotin-TSSG-K(Ac)-I-Nle-S-K(MR121)-$NH_2$ | SEQ ID NO: 15 |
| Sirt3/ Acetyl CoA Synthase 2 | Biotin-TSSG-K(Ac)-I-Nle-S-K(5-TMR)-$NH_2$ | SEQ ID NO: 16 |
| Sirt3/ Acetyl CoA Synthase 2 | Biotin-PSTSSG-K(Ac)-I-Nle-SS-K(MR121)-$NH_2$ | SEQ ID NO: 17 |
| Sirt3/ Acetyl CoA Synthase 2 | Biotin-PSTSSG-K(Ac)-I-Nle-SS-K(5-TMR)-$NH_2$ | SEQ ID NO: 18 |
| Sirt3/ Histone H4 | Biotin-SGSG-K(Ac)-GGS-K(MR121)-$NH_2$ | SEQ ID NO: 19 |
| Sirt3/ Histone H4 | Biotin-SGSG-K(Ac)-GGS-K(5-TMR)-$NH_2$ | SEQ ID NO: 20 |
| Sirt3/ Histone H4 | Biotin-GSGGA-K(Ac)-SHS-K(MR121)-$NH_2$ | SEQ ID NO: 21 |
| Sirt3/ Histone H4 | Biotin-GSGGA-K(Ac)-SHS-K(5-TMR)-$NH_2$ | SEQ ID NO: 22 |
| Sirt3/ Histone H4 | Biotin-GASSHS-K(Ac)-VL-K(MR121)-$NH_2$ | SEQ ID NO: 23 |
| Sirt3/ Histone H4 | Biotin-GASSHS-K(Ac)-VL-K(5-TMR)-$NH_2$ | SEQ ID NO: 24 |

The invention describes a peptide substrate for use in determining the activity of an acetyltransferase or deacetylase using fluorescence polarization. The peptide substrate comprises a bulky group (or high molecular weight group) or binding site for a bulky group, a fluorophore and a lysine (for use as a substrate for an acetyltransferase) or an acetylated lysine (for use as a substrate for a deacetylase) located between the bulky group and the fluorophore. In

certain embodiments the peptide substrate may have the general formula $X_1$ - $X_2$ - $X_3$ - $X_4$ - $X_5$ - $X_6$ - $X_7$, wherein $X_1$ is from 0-50 amino acid residues, $X_2$ is a binding site for a bulky group or a bulky group, $X_3$ is from 0-50 amino acids, $X_4$ is a lysine or an acetylated lysine, $X_5$ is from 0-50 amino acids, $X_6$ is an amino acid modified with a fluorophore, $X_7$ is from 0-50 amino acid residues. In certain embodiments, $X_1$, $X_3$, $X_5$ and/or $X_7$ are amino acids other than lysine or arginine. In certain embodiments, the bulky group may be an amino acid sequence, a binding site for a bulky group, an amino acid residue modified with a bulky group, or an amino acid modified with a binding site for a bulky group. In certain embodiments, the peptide substrate has the general formula $X_1$ - X2 - $X_3$ - $X_4$ - $X_5$ - $X_6$ - $X_7$, wherein $X_1$ is from 0-10, 0-5 or 0-2 amino acids, $X_2$ is an amino acid modified with a binding site for a bulky group, such as biotin, $X_3$ is from 1-20, 1-10, or 1-5 amino acids, $X_4$ is a lysine or an acetylated lysine, $X_5$ is from 1-10, 1-5, or 2-5 amino acids, $X_6$ is an amino acid modified with a fluorophore, $X_7$ is from 0-10, 0-5, or 0-2 amino acid residues.

The invention describes a peptide substrate for use in determining the activity of an acetyltransferase or deacetylase using fluorescence polarization wherein the sequence of the peptide substrate may be based on, or derived from, any substrate for an acetyltransferase or deacetylase enzyme. In exemplary embodiments, the sequence of the peptide substrate is based on PGC1 alpha, alpha tubulin, Acetyl CoA synthetase 2, or a histone, such as, for example, histone H4, or portions thereof. In one embodiment, the peptide substrate is based on a portion of p53 and comprises the amino acid sequence $EEX_1GQSTSSHSX_2X_3STEGX_4EE$ (SEQ ID NO: 25), wherein $X_1$ is bulky group or a binding site for a bulky group, $X_2$ is lysine or an acetylated lysine, $X_3$ is an amino acid other than lysine or arginine, and $X_4$ is an amino acid modified with a fluorophore. In another embodiment, the peptide substrate may be based on a portion of pGC1 alpha and comprises the amino acid sequence $X_1NPAIVX_2TENSX_3$ (SEQ ID NO: 26) or $X_1HLQAX_2PTTLSX_3$ (SEQ ID NO: 27), wherein $X_1$ is a bulky group or a binding site for a bulky group, $X_2$ is lysine or an acetylated lysine, and $X_3$ is an amino acid modified with a fluorophore. In one embodiment, the peptide substrate is based on a portion of alpha tubulin and comprises the amino acid sequence $X_1PSDX_2TIGGX_3$ (SEQ ID NO: 28), wherein $X_1$ is a bulky group or a binding site for a bulky group, $X_2$ is lysine or an acetylated lysine, and $X_3$ is an amino acid modified with a fluorophore; or comprises the amino acid sequence $X_1QX_2PSDX_3TIGGX_4$ (SEQ ID NO: 29), wherein $X_1$ is a bulky group or a binding site for a bulky group, $X_2$ is an amino acid other than lysine or arginine, $X_3$ is lysine or an acetylated lysine, and $X_4$ is an amino acid modified with a fluorophore. In one embodiment, the peptide substrate is based on a portion of acetyl CoA synthetase 2 and comprises the amino acid sequence $X_1GX_2IMX_3$ (SEQ ID NO: 30), wherein $X_1$ is a bulky group or a binding site for a bulky group , $X_2$ is lysine or an acetylated lysine, and $X_3$ is an amino acid modified with a fluorophore; or comprises the amino acid sequence $X_1SSGX_2IX_3SX_4$ (SEQ ID NO: 31) or $X_1STSSGX_2IX_3SSX_4$ (SEQ ID NO: 32), wherein $X_1$ is a bulky group or a binding site for a bulky group, $X_2$ is lysine or an acetylated lysine, $X_3$ is an amino acid other than lysine, or arginine, and $X_4$ is an amino acid modified with a fluorophore. In one embodiment, the peptide substrate is based on a portion of histone H4 and comprises the amino acid sequence $X_1GSGX_2GGSX_3$ (SEQ ID NO: 33), $X_1SGGAX_2SHSX_3$ (SEQ ID NO: 34), or $X_1ASSHSX_2VLX_3$ (SEQ ID NO: 35), wherein $X_1$ is a bulky group or a binding site for a bulky group, $X_2$ is lysine or an acetylated lysine, and $X_3$ is an amino acid modified with a fluorophore.

The invention describes a peptide substrate for use in determining the activity of an acetyltransferase or deacetylase using fluorescence polarization wherein the substrate comprises one or more of SEQ ID NOs: 1-24.

The methods described herein utilize a cleavage reagent that cleaves peptide substrates containing non-acetylated lysine residues but will not cleave peptide substrates containing acetylated lysine residues. The cleavage reagent may be a chemical or enzymatic reagent. In an exemplary embodiment, the cleavage reagent is a protease, such as, for example, a protease that cleaves at or near a lysine residue. Exemplary proteases included, for example, lysylendopeptidase, endoproteinase, Lys-C, plasmin, calpain, or trypsin.

In certain embodiments, the methods described herein are carried out under conditions which permit acetylation or deacetylation of the peptide substrate by an acetyltransferase or deacetylase, respectively.

In certain embodiments, the substrate peptide pool comprises a plurality of copies of one or more substrate peptides. In an exemplary embodiment, a substrate peptide pool comprises a plurality of copies of the same peptide substrate. Such peptide substrate pools may comprise the peptide substrate free floating in solution or attached to a solid surface such as a plate, bead, filter, etc. Combinations of free floating and anchored peptide substrate molecules may also be used in accordance with the methods described herein.

In certain embodiments, the methods described herein may be carried out in a single reaction vessel without the need to remove reagents from the reaction mixture (e.g., a homogenous assay). In various embodiments, the components of the reactions described herein may be added sequentially or simultaneously. For example, it is possible to add the cleavage reagent concurrently with, or subsequent to, exposure of the peptide substrate to the acetyltransferase or deacetylase.

In certain embodiments, the invention provides a method for identifying a compound that modulates the activity of an acetyltransferase or deacetylase. The methods may involve comparing the activity of an acetyltransferase or deacetylase in the presence of a test compound to the activity of the acetyltransferase or deacetylase in a control reaction. The control reaction may simply be a duplicate reaction in which the test compound is not included. Alternatively, the control reaction may be a duplicate reaction in the presence of a compound having a known effect on the acetyltransferase or deacetylase activity (e.g., an activator, an inhibitor, or a compound having no effect on enzyme activity).

Due to the flexibility available in designing peptide substrates for the fluorescence polarization based methods described herein, it is possible to optimize the peptide substrates to provide a low apparent Km thus permitting a lower concentration of substrate to be used in association with the methods. Table 3 shown below provides the Km values for a variety of sirtuin peptide substrates provided in accordance with the methods described herein in comparison to the Km values of several published or commercially available sirtuin assays. Accordingly, in certain embodiments, the substrate peptides for use in accordance with the methods described herein may be optimized to provide a low apparent Km.

Table 3. Km comparisons for various sirtuin assays.

| **Reference** | **Assay** | **Enzyme** | **Peptide substrate (*FL with fluorescent label)** | **Peptide Km (uM)** | **NAD Km (uM)** |
|---|---|---|---|---|---|
| Biomol (Fluor de Lys)[1] | Fluorescent | Sirt1 | p53 (aa 379-382) *FL | 64 | 558 |
| | Fluorescent | Sirt2 | p53 (aa 317-320) *FL | 186 | 547 |
| | Fluorescent | Sirt3 | p53 (aa 317-320) *FL | 32 | 2034 |
| [2]Kaeberlein et al. | Nicotinamide release | Sirt1 | p53 (aa 368-386) no FL | 10.3 | 132.5 |
| | Nicotinamide release | Sirt1 | p53 (aa 368-386) *FL | 87.6 | 192 |
| [3]Marcotte et al. | FRET | Sirt1 | p53 20 mer (aa 372-387) *FL | ND | 90 |
| | FRET | Sirt2 | p53 20 mer (aa 372-387) *FL | ND | 42 |
| [4]McDonagh et al. | Nicotinamide release | Sirt1 | p53 19 mer (aa 368-386) no FL | 10.3 | 133 |
| | | | | | |
| Present invention | Fluorescent polarization | Sirt1 | SEQ ID NO:2 | 7.8 | 464 |
| Present invention | Fluorescent polarization | Sirt2 | SEQ ID NO: 9 | 50 | 37 |
| Present invention | Fluorescent polarization | Sirt3 | SEQ ID NO: 23 | ND | 50 |

[1]Biomol (Fluor de Lys) as described in product literature for SIRT1 Fluorimetric Drug Discovery Kit (AK-555), SIRT2 Fluorimetric Drug Discovery Kit (AK-556), SIRT3 Fluorimetric Drug Discovery Kit (AK-557) (Biomol International, Plymouth Meeting, PA);
[2]Kaeberlein et al., JBC, 280 (17), 17038,2005; [3]Marcotte et al., Anal. Biochem., 332, 90, 2004; [4]McDonagh et al., Methods, 36, 346, 2005.

In certain embodiments, the invention provides methods for screening for compounds that modulate activity of an acetyltransferase or deacetylase. In certain embodiments, the methods described herein may be used to identify a test compound that decreases or increases acetylase or deacetylase activity by at least about 10%, 25%, 50%, 75%, 80%, 90%, or 100%, or more, relative to the absence of the test compound.

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid

phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. See Lam, Anticancer Drug Des. 12, 145, 1997.

Methods for the synthesis of molecular libraries are well known in the art (see, for example, DeWitt et al., Proc. Natl. Acad. Sci. U.S.A. 90, 6909, 1993; Erb et al. Proc. Natl. Acad. Sci. U.S.A. 91, 11422, 1994; Zuckermann et al., J Med Chem. 37,2678, 1994; Cho et al., Science 261, 1303, 1993; Carell et al., Angew. Chem. Int. Ed Engl. 33, 2059, 1994; Carell et al., Angew. Chem. Int. Ed. Engl. 33, 2061; Gallop et al., J. Med Chem. 37, 1233, 1994). Libraries of compounds can be presented in solution (see, e.g., Houghten, BioTechniques 13, 412-421, 1992), or on beads (Lam, Nature 354, 82-84, 1991), chips (Fodor, Nature 364, 555-556, 1993), bacteria or spores (Ladner, U.S. Pat. No. 5,223,409), plasmids (Cull et al., Proc. Natl. Acad Sci. U.S.A. 89, 1865-1869, 1992), or phage (Scott & Smith, Science 249, 386-390, 1990; Devlin, Science 249, 404-406, 1990); Cwirla et al., Proc. Natl. Acad. Sci. 97, 6378-6382, 1990; Felici, J. Mol. Biol. 222, 301-310, 1991; and Ladner, U.S. Pat. No. 5,223,409).

Test compounds can be screened for the ability to modulate acetyltransferase or deacetylase activity using high throughput screening. Using high throughput screening, many discrete compounds can be tested in parallel so that large numbers of test compounds can be quickly screened. The most widely established techniques utilize 96-well microtiter plates. In addition to the plates, many instruments, materials, pipettors, robotics, plate washers, and plate readers are commercially available to fit the 96-well format.

Alternatively, free format assays, or assays that have no physical barrier between samples, can be used. Assays involving free formats are described, for example, in Jayawickreme et al., Proc. Natl. Acad. Sci. U.S.A. 19, 1614-18 (1994); Chelsky, "Strategies for Screening Combinatorial Libraries: Novel and Traditional Approaches," reported at the First Annual Conference of The Society for Biomolecular Screening in Philadelphia, Pa. (Nov. 7-10, 1995); and Salmon et al., Molecular Diversity 2, 57-63 (1996). Another high throughput screening method is described in Beutel et al., U.S. Pat. No. 5,976,813.

Compounds that activate or inhibit the acetyltransferase or deacetylase activity, which can be selected according to the method for screening of the present invention, are useful as candidate compounds for antimicrobial substances, anti-cancer agents, and a variety of other uses. For example, compounds that activate a sirtuin deacetylase protein may be useful for increasing the lifespan of a cell, and treating and/or preventing a wide variety of diseases and disorders including, for example, diseases or disorders related to aging or stress, diabetes, obesity, neurodegenerative diseases, chemotherapeutic induced neuropathy, neuropathy associated with an ischemic event, ocular diseases and/or disorders, cardiovascular disease, blood clotting disorders, inflammation, and/or flushing, etc. In other embodiments, sirtuin deacetylase inhibitors may be useful for a variety of therapeutic applications including, for example, increasing cellular sensitivity to stress, increasing apoptosis, treatment of cancer, stimulation of appetite, and/or stimulation of weight gain, etc.

The invention describes a peptide substrate for use in determining the activity of a sirtuin using fluorescence polarization wherein the peptide substrate comprises the amino acid sequence GQSTSSHSK(Ac)NleSTEG (SEQ ID NO: 36), wherein the peptide substrate is labeled with a fluorophore and biotin. In various embodiments, the fluorophore maybe MR121 or 5TMR. In certain embodiments, the peptide substrate is a substrate for SIRT1 or SIRT3. In certain embodiments, the peptide substrate is labeled at the N-terminus with biotin and at the C-terminus with the fluorophore.

The invention describes a peptide substrate for use in determining the activity of a sirtuin using fluorescence polarization wherein the peptide substrate comprises the amino acid sequence GASSHSK(Ac)VLK (SEQ ID NO: 37), wherein the peptide substrate is labeled with a fluorophore and biotin. In various embodiments, the fluorophore may be MR121 or 5TMR. In certain embodiments, the peptide substrate comprises the amino acid sequence Biotin-GASSHS-K(Ac)-VL-K(MR121)-$NH_2$ (SEQ ID NO: 23) or Biotin-GASSHS-K(Ac)-VL-K(5-TMR)-$NH_2$ (SEQ ID NO: 24). In certain embodiments, the peptide substrate is a substrate for SIRT1 or SIRT3. In certain embodiments, the peptide substrate is labeled at the N-terminus with biotin and at the C-terminus with the fluorophore.

The invention describes a peptide substrate for use in determining the activity of a sirtuin using fluorescence polarization wherein the peptide substrate comprises the amino acid sequence EEKGQSTSSHSK(Ac)NleSTEGKEE (SEQ ID NO: 38), wherein the peptide substrate is labeled with a fluorophore and biotin. In various embodiments, the fluorophore may be MR121 or 5TMR. In certain embodiments, the peptide substrate comprises the amino acid sequence Ac-EE-K(biotin)-GQSTSSHS-K(Ac)-Nle-STEG-K(MR121)-EE-$NH_2$ (SEQ ID NO: 1) or Ac-EE-K(biotin)-GQSTSSHS-K (Ac)-Nle-STEG-K(5-TMR)-EE-$NH_2$ (SEQ ID NO: 2). In certain embodiments, the peptide substrate is a substrate for SIRT1 or SIRT3. In certain embodiments, the peptide substrate is labeled at the N-terminal lysine with biotin and at the C-terminal lysine with the fluorophore.

The invention describes a peptide substrate determining the activity of an acetyltransferase or deacetylase using fluorescence polarization wherein the peptide substrate comprises a bulky group or a binding site for a bulky group, a fluorophore, and a lysine or acetylated lysine located between the bulky group, or the binding site for a bulky group and the fluorophore, with the *proviso* that the peptide substrate does not comprise (a) the amino acid sequence

GQSTSSHSK(Ac)NleSTEG (SEQ ID NO: 36), wherein the peptide substrate of SEQ ID NO: 36 is labeled with a fluorophore and biotin; (b) the amino acid sequence GQSTSSHSK(Ac)NleSTEG (SEQ ID NO: 36), wherein the peptide substrate is labeled at the N-terminus with biotin and at the C-terminus with the fluorophore; (c) the amino acid sequence GQSTSSHSK(Ac)NleSTEG (SEQ ID NO: 36), wherein the peptide substrate is labeled at the N-terminus with biotin and at the C-terminus with MR121; and/or (d) the amino acid sequence GQSTSSHSK(Ac)NleSTEG (SEQ ID NO: 36), wherein the peptide substrate is labeled at the N-terminus with biotin and at the C-terminus with 5TMR.

The invention describes a peptide substrate for use in determining the activity of an acetyltransferase or deacetylase using fluorescence polarization wherein the peptide substrate comprises a bulky group or a binding site for a bulky group, a fluorophore, and a lysine or acetylated lysine located between the bulky group, or the binding site for a bulky group and the fluorophore, with the *proviso* that the peptide substrate does not comprise (a) the amino acid sequence GASSHSK(Ac)VLK (SEQ ID NO: 37), wherein the peptide substrate of SEQ ID NO: 37 is labeled with a fluorophore and biotin; (b) the amino acid sequence Biotin-GASSHS-K(Ac)-VL-K(MR121)-$NH_2$ (SEQ ID NO: 23); and/or (c) the amino acid sequence Biotin-GASSHS-K(Ac)-VL-K(5-TMR)-$NH_2$ (SEQ ID NO: 24).

The invention describes a peptide substrate for use in determining the activity of an acetyltransferase or deacetylase using fluorescence polarization wherein the peptide substrate comprises a bulky group or a binding site for a bulky group, a fluorophore, and a lysine or acetylated lysine located between the bulky group, or the binding site for a bulky group and the fluorophore, with *the proviso* that the peptide substrate does not comprise (a) the amino acid sequence EEKGQSTSSHSK(Ac)NleSTEGKEE (SEQ ID NO: 38), wherein the peptide substrate of SEQ ID NO: 38 is labeled with a fluorophore and biotin; (b) the amino acid sequence Ac-EE-K(biotin)-GQSTSSHS-K(Ac)-Nle-STEG-K(MR121)-EE-$NH_2$ (SEQ ID NO: 1); and/or (c) the amino acid sequence Ac-EE-K(biotin)-GQSTSSHS-K(Ac)-Nle-STEG-K(5-TMR)-EE-$NH_2$ (SEQ ID NO: 2).

The invention describes a method for determining the activity of a sirtuin protein, comprising: (a) exposing a peptide substrate to a sirtuin protein, wherein the peptide substrate comprises the amino acid sequence GQSTSSHSK (Ac)NleSTEG (SEQ ID NO: 36) and is labeled with a fluorophore and biotin; (b) cleaving the peptide substrate with trypsin; and (c) observing the fluorescence polarization value of the peptide substrate, wherein a decrease in the fluorescence polarization value of the peptide substrate is indicative of sirtuin activity. In certain embodiments, the fluorophore of the peptide substrate may be MR121 or 5TMR. In certain embodiments, the sirtuin protein is a SIRT1 or SIRT3 protein. In certain embodiments, the peptide substrate is labeled at the N-terminus with biotin and at the C-terminus with the fluorophore. In certain embodiments, the method further comprises contacting the peptide substrate with streptavidin.

In one embodiment, the invention provides a method for determining the activity of a sirtuin protein, comprising: (a) exposing a peptide substrate to a sirtuin protein, wherein the peptide substrate comprises the amino acid sequence GASSHSK(Ac)VLK (SEQ ID NO: 37) and is labeled with a fluorophore and biotin; (b) cleaving the peptide substrate with trypsin; and (c) observing the fluorescence polarization value of the peptide substrate, wherein a decrease in the fluorescence polarization value of the peptide substrate is indicative of sirtuin activity. In certain embodiments, the fluorophore of the peptide substrate may be MR121 or 5TMR. In certain embodiments, the peptide substrate comprises the amino acid sequence Biotin-GASSHS-K(Ac)-VL-K(NM121)-$NH_2$ (SEQ ID NO: 23) or Biotin-GASSHS-K(Ac)-VL-K (5-TMR)-$NH_2$ (SEQ ID NO: 24). In certain embodiments, the sirtuin protein is a SIRT1 or SIRT3 protein. In certain embodiments, the peptide substrate is labeled at the N-terminus with biotin and at the C-terminus with the fluorophore. In certain embodiments, the method further comprises contacting the peptide substrate with streptavidin.

In one embodiment, the invention provides a method for determining the activity of a sirtuin protein, comprising: (a) exposing a peptide substrate to a sirtuin protein, wherein the peptide substrate comprises the amino acid sequence EEKGQSTSSHSK(Ac)NleSTEGKEE (SEQ ID NO: 38) and is labeled with a fluorophore and biotin; (b) cleaving the peptide substrate with trypsin; and (c) observing the fluorescence polarization value of the peptide substrate, wherein a decrease in the fluorescence polarization value of the peptide substrate is indicative of sirtuin activity. In certain embodiments, the fluorophore of the peptide substrate may be MR121 or 5TMR. In certain embodiments, the peptide substrate comprises the amino acid sequence Ac-EE-K(biotin)-GQSTSSHS-K(Ac)-Nle-STEG-K(MR121)-EE-$NH_2$ (SEQ ID NO: 1) or Ac-EE-K(biotin)-GQSTSSHS-K(Ac)-Nle-STEG-K(5-TNM)-EE-$NH_2$ (SEQ ID NO: 2). In certain embodiments, the sirtuin protein is a SIRT1 or SIRT3 protein. In certain embodiments, the peptide substrate is labeled at the N-terminus with biotin and at the C-terminus with the fluorophore. In certain embodiments, the method further comprises contacting the peptide substrate with streptavidin.

In one embodiment, the invention provides a method for identifying a modulator of a sirtuin protein, comprising: (a) exposing a peptide substrate to a sirtuin protein in the presence of a test compound, wherein the peptide substrate comprises the amino acid sequence GQSTSSHSK(Ac)NleSTEG (SEQ ID NO: 36) and is labeled with a fluorophore and biotin; (b) cleaving the peptide substrate with trypsin; and (c) observing the fluorescence polarization value of the peptide substrate, wherein a change in the fluorescence polarization value of the peptide substrate in the presence of the test compound as compared to a control is indicative of a compound that modulates the activity of the sirtuin. In certain embodiments, the fluorophore of the peptide substrate may be MR121 or 5TMR. In certain embodiments, the sirtuin protein is a SIRT1 or SIRT3 protein. In certain embodiments, the peptide substrate is labeled at the N-terminus with

biotin and at the C-terminus with the fluorophore. In certain embodiments, the method further comprises contacting the peptide substrate with streptavidin.

In one embodiment, the invention provides a method for identifying a modulator of a sirtuin protein, comprising: (a) exposing a peptide substrate to a sirtuin protein in the presence of a test compound, wherein the peptide substrate comprises the amino acid sequence GASSHSK(Ac)VLK (SEQ ID NO: 37) and is labeled with a fluorophore and biotin; (b) cleaving the peptide substrate with trypsin; and (c) observing the fluorescence polarization value of the peptide substrate, wherein a change in the fluorescence polarization value of the peptide substrate in the presence of the test compound as compared to a control is indicative of a compound that modulates the activity of the sirtuin. In certain embodiments, the fluorophore of the peptide substrate may be MR121 or 5TMR. In certain embodiments, the peptide substrate comprises the amino acid sequence Biotin-GASSHS-K(Ac)-VL-K(MR121)-$NH_2$ (SEQ ID NO: 23) or Biotin-GASSHS-K(Ac)-VL-K(5-TMR)-$NH_2$ (SEQ ID NO: 24). In certain embodiments, the sirtuin protein is a SIR1 or SIRT3 protein. In certain embodiments, the peptide substrate is labeled at the N-terminus with biotin and at the C-terminus with the fluorophore. In certain embodiments, the method further comprises contacting the peptide substrate with streptavidin.

In one embodiment, the invention provides a method for identifying a modulator of a sirtuin protein, comprising: (a) exposing a peptide substrate to a sirtuin protein in the presence of a test compound, wherein the peptide substrate comprises the amino acid sequence EEKGQSTSSHSK(Ac)NleSTEGKEE (SEQ ID NO: 38) and is labeled with a fluorophore and biotin; (b) cleaving the peptide substrate with trypsin; and (c) observing the fluorescence polarization value of the peptide substrate, wherein a change in the fluorescence polarization value of the peptide substrate in the presence of the test compound as compared to a control is indicative of a compound that modulates the activity of the sirtuin. In certain embodiments, the fluorophore of the peptide substrate may be MR121 or 5TMR. In certain embodiments, the peptide substrate comprises the amino acid sequence Ac-EE-K(biotin)-GQSTSSHS-K(Ac)-Nle-STEG-K(MR121)-EE-$NH_2$ (SEQ ID NO: 1) or Ac-EE-K(biotin)-GQSTSSHS-K(Ac)-Nle-STEG-K(5-TMR)-EE-$NH_2$ (SEQ ID NO: 2). In certain embodiments, the sirtuin protein is a SIRT1 or SIRT3 protein. In certain embodiments, the peptide substrate is labeled at the N-terminus with biotin and at the C-terminus with the fluorophore. In certain embodiments, the method further comprises contacting the peptide substrate with streptavidin.

In certain embodiments, the invention provides a method for determining the activity of a deacetylase, comprising: (i) contacting a peptide substrate pool with a deacetylase, wherein members of said peptide substrate pool comprise a fluorophore and at least one acetylated lysine residue; (ii) contacting the peptide substrate pool with a reagent that cleaves members of the peptide substrate pool having non-acetylated lysine residues; and (iii) determining the fluorescence polarization value of the peptide substrate pool, wherein a decrease in the fluorescence polarization value of the peptide substrate pool is indicative of deacetylase activity, with the *proviso* that (a) the method does not comprise determining the activity of a sirtuin protein by exposing the sirtuin protein to a peptide substrate comprising the amino acid sequence GQSTSSHSK(Ac)NleSTEG (SEQ ID NO: 36), wherein said peptide substrate is labeled with a fluorophore and biotin; (b) the method does not comprise determining the activity of a sirtuin protein by exposing the sirtuin protein to a peptide substrate comprising the amino acid sequence GASSHSK(Ac)VLK (SEQ ID NO: 37), wherein said peptide substrate is labeled with a fluorophore and biotin; (c) the method does not comprise determining the activity of a sirtuin protein by exposing the sirtuin protein to a peptide substrate comprising the amino acid sequence EEKGQSTSSHSK (Ac)NleSTEGKEE (SEQ ID NO: 38), wherein said peptide substrate is labeled with a fluorophore and biotin, and/or (d) the method does not comprise determining the activity of a sirtuin protein by exposing the sirtuin protein to a peptide substrate comprising one or more of the following: SEQ ID NO: 1, 2, 23, or 24.

In certain embodiments, the invention provides a method for determining the activity of a deacetylase, comprising: (i) contacting a peptide substrate pool with a deacetylase, wherein members of said peptide substrate pool comprise a fluorophore and at least one acetylated lysine residue; (ii) contacting the peptide substrate pool with a reagent that cleaves members of the peptide substrate pool having non-acetylated lysine residues; and (iii) determining the fluorescence polarization value of the peptide substrate pool, wherein a decrease in the fluorescence polarization value of the peptide substrate pool is indicative of deacetylase activity, with the *proviso* that (a) the deacetylase is not a sirtuin protein, (b) the deacetylase is not human SIRT2, (c) the deacetylase is not a SIRT1 protein, (d) the deacetylase is not human SIRT3, (e) the deacetylase is not a SIRT3 protein, (f) the deacetylase is not a SIRT1 or SIRT3 protein, (g) the peptide substrate is not Ac-EE-K(biotin)-GQSTSSHSK(Ac)NleSTEG-K(MR121)-EE-$NH_2$ (SEQ ID NO: 1), (h) the peptide substrate is not Ac-EE-K(biotin)-GQSTSSHSK(Ac)NleSTEG-K(5TMR)-EE-NH2 (SEQ ID NO: 2), (i) the peptide substrate is not Biotin-GASSHSK(Ac)VLK(MR121) (SEQ ID NO: 23), (j) the peptide substrate is not GQSTSSHSK(Ac)NleSTEG (SEQ ID NO: 36) wherein the peptide substrate is labeled with a fluorophore and biotin, (k) the peptide substrate is not GASSHSK(Ac)VLK (SEQ ID NO: 37) wherein the peptide substrte is labeled with a fluorophore and biotin, and/or (l) the peptide substrate is not EEKGQSTSSHSK(Ac)NleSTEGKEE (SEQ ID NO: 38) wherein the peptide substrate is labeled with a fluorophore and biotin.

In certain embodiments, the invention provides a method for identifying a compound that modulates a deacetylase, comprising: (i) contacting a peptide substrate pool with a deacetylase in the presence of a test compound, wherein

members of said peptide substrate pool comprise a fluorophore and at least one acetylated lysine residue; (ii) contacting the peptide substrate pool with a compound that cleaves members of the peptide substrate pool having non-acetylated lysine residues; and (iii) determining the fluorescence polarization value of the peptide substrate pool, wherein a change in the fluorescence polarization value of the peptide substrate pool in the presence of the test compound as compared to a control is indicative of a compound that modulates the activity of the deacetylase, with the *proviso* that (a) the method does not comprise identifying a modulator of a sirtuin protein by exposing the sirtuin protein to a peptide substrate in the presence of a test compound, wherein the peptide substrate comprises the amino acid sequence GQSTSSHSK(Ac)NleSTEG (SEQ ID NO: 36) and is labeled with a fluorophore and biotin; (b) the method does not comprise identifying a modulator of a sirtuin protein by exposing the sirtuin protein to a peptide substrate in the presence of a test compound, wherein the peptide substrate comprises the amino acid sequence GASSHSK(Ac)VLK (SEQ ID NO: 37) and is labeled with a fluorophore and biotin; (c) the method does not comprise identifying a modulator of a sirtuin protein by exposing the sirtuin protein to a peptide substrate in the presence of a test compound, wherein the peptide substrate comprises the amino acid sequence EEKGQSTSSHSK(Ac)NleSTEGKEE (SEQ ID NO: 38) and is labeled with a fluorophore and biotin, and/or (d) the method does not comprise identifying a modulator of a sirtuin protein by exposing the sirtuin protein to a peptide substrate in the presence of a test compound, wherein the peptide substrate comprises one or more of the following: SEQ ID NO: 1, 2, 23, or 24.

In certain embodiments, the invention provides a method for identifying a compound that modulates a deacetylase, comprising: (i) contacting a peptide substrate pool with a deacetylase in the presence of a test compound, wherein members of said peptide substrate pool comprise a fluorophore and at least one acetylated lysine residue; (ii) contacting the peptide substrate pool with a compound that cleaves members of the peptide substrate pool having non-acetylated lysine residues; and (iii) determining the fluorescence polarization value of the peptide substrate pool, wherein a change in the fluorescence polarization value of the peptide substrate pool in the presence of the test compound as compared to a control is indicative of a compound that modulates the activity of the deacetylase, with the *proviso* that (a) the deacetylase is not a sirtuin protein, (b) the deacetylase is not human SIRT1, (c) the deacetylase is not a SIRT1 protein, (d) the deacetylase is not human SIRT3, (e) the deacetylase is not a SIRT3 protein, (f) the deacetylase is not a SIRT1 or SIRT3 protein, (g) the peptide substrate is not Ac-EE-K(biotin)-GQSTSSHSK(Ac)NleSTEG-K(MR121)-EE-$NH_2$ (SEQ ID NO: 1), (h) the peptide substrate is not Ac-EE-K(biotin)-GQSTSSHSK(Ac)NleSTEG-K(5TMR)-EE-NH2 (SEQ ID NO: 2), (i) the peptide substrate is not Biotin-GASSHSK(Ac)VLK(MR121) (SEQ ID NO: 23), (j) the peptide substrate is not GQSTSSHSK(Ac)NleSTEG (SEQ ID NO: 36) wherein the peptide substrate is labeled with a fluorophore and biotin, (k) the peptide substrate is not GASSHSK(Ac)VLK (SEQ ID NO: 37) wherein the peptide substrte is labeled with a fluorophore and biotin, and/or (l) the peptide substrate is not EEKGQSTSSHSK(Ac)NleSTEGKEE (SEQ ID NO: 38) wherein the peptide substrate is labeled with a fluorophore and biotin.

In one embodiment, the invention provides a method for determining the activity of an enzyme that modulates acetylation of a peptide substrate, comprising (i) contacting a peptide substrate pool with an enzyme that modulates acetylation; (ii) contacting the peptide substrate pool with a cleavage reagent; and (iii) determining the fluorescence polarization value of the peptide substrate pool, wherein a change in the fluorescence polarization value of the peptide substrate pool is indicative of activity of the enzyme that modulates acetylation, with the *proviso* that (a) the method does not comprise determining the activity of a sirtuin protein by exposing the sirtuin protein to a peptide substrate comprising the amino acid sequence GQSTSSHSK(Ac)NleSTEG (SEQ ID NO: 36), wherein said peptide substrate is labeled with a fluorophore and biotin; (b) the method does not comprise determining the activity of a sirtuin protein by exposing the sirtuin protein to a peptide substrate comprising the amino acid sequence GASSHSK(Ac)VLK (SEQ ID NO: 37), wherein said peptide substrate is labeled with a fluorophore and biotin; (c) the method does not comprise determining the activity of a sirtuin protein by exposing the sirtuin protein to a peptide substrate comprising the amino acid sequence EEKGQSTSSHSK(Ac)NleSTEGKEE (SEQ ID NO: 38), wherein said peptide substrate is labeled with a fluorophore and biotin, and/or (d) the method does not comprise determining the activity of a sirtuin protein by exposing the sirtuin protein to a peptide substrate comprising one or more of the following: SEQ ID NO: 1, 2, 23, or 24.

In one embodiment, the invention provides a method for determining the activity of an enzyme that modulates acetylation of a peptide substrate, comprising (i) contacting a peptide substrate pool with an enzyme that modulates acetylation; (ii) contacting the peptide substrate pool with a cleavage reagent; and (iii) determining the fluorescence polarization value of the peptide substrate pool, wherein a change in the fluorescence polarization value of the peptide substrate pool is indicative of activity of the enzyme that modulates acetylation, with the *proviso* that (a) the enzyme that modulates acetylation is not a sirtuin protein, (b) the enzyme that modulates acetylation is not human SIRT1, (c) the enzyme that modulates acetylation is not a SIRT1 protein, (d) the enzyme that modulates acetylation is not human SIRT3, (e) the enzyme that modulates acetylation is not a SIRT3 protein, (f) the enzyme that modulates acetylation is not a SIRT1 or SIRT3 protein, (g) the peptide substrate is not Ac-EE-K(biotin)-GQSTSSHSK(Ac)NleSTEG-K(MR121)-EE-$NH_2$ (SEQ ID NO: 1), (h) the peptide substrate is not Ac-EE-K(biotin)-GQSTSSHSK(Ac)NleSTEG-K(5TMR)-EE-NH2 (SEQ ID NO: 2), (i) the peptide substrate is not Biotin-GASSHSK(Ac)VLK(MR121) (SEQ ID NO: 23), (j) the peptide substrate is not GQSTSSHSK(Ac)NleSTEG (SEQ ID NO: 36) wherein the peptide substrate is labeled

with a fluorophore and biotin, (k) the peptide substrate is not GASSHSK(Ac)VLK (SEQ ID NO: 37) wherein the peptide substrte is labeled with a fluorophore and biotin, and/or (1) the peptide substrate is not EEKGQSTSSHSK(Ac)NleSTEGKEE (SEQ ID NO: 38) wherein the peptide substrate is labeled with a fluorophore and biotin.

A method for identifying a compound that modulates the activity of an enzyme that modulates acetylation of a peptide substrate, comprising (i) contacting a peptide substrate pool with an enzyme that modulates acetylation in the presence of a test compound; (ii) contacting the peptide substrate pool with a cleavage reagent; and (iii) determining the fluorescence polarization value of the peptide substrate pool, wherein a change in the fluorescence polarization value of the peptide substrate pool in the presence of the test compound is indicative of a compound that modulates the activity of the enzyme that modulates acetylation, with the *proviso* that that (a) the method does not comprise identifying a modulator of a sirtuin protein by exposing the sirtuin protein to a peptide substrate in the presence of a test compound, wherein the peptide substrate comprises the amino acid sequence GQSTSSHSK(Ac)NleSTEG (SEQ ID NO: 36) and is labeled with a fluorophore and biotin; (b) the method does not comprise identifying a modulator of a sirtuin protein by exposing the sirtuin protein to a peptide substrate in the presence of a test compound, wherein the peptide substrate comprises the amino acid sequence GASSHSK(Ac)VLK (SEQ ID NO: 37) and is labeled with a fluorophore and biotin; (c) the method does not comprise identifying a modulator of a sirtuin protein by exposing the sirtuin protein to a peptide substrate in the presence of a test compound, wherein the peptide substrate comprises the amino acid sequence EEKGQSTSSHSK(Ac)NleSTEGKEE (SEQ ID NO: 38) and is labeled with a fluorophore and biotin, and/or (d) the method does not comprise identifying a modulator of a sirtuin protein by exposing the sirtuin protein to a peptide substrate in the presence of a test compound, wherein the peptide substrate comprises one or more of the following: SEQ ID NO: 1, 2, 23, or 24.

A method for identifying a compound that modulates the activity of an enzyme that modulates acetylation of a peptide substrate, comprising (i) contacting a peptide substrate pool with an enzyme that modulates acetylation in the presence of a test compound; (ii) contacting the peptide substrate pool with a cleavage reagent; and (iii) determining the fluorescence polarization value of the peptide substrate pool, wherein a change in the fluorescence polarization value of the peptide substrate pool in the presence of the test compound is indicative of a compound that modulates the activity of the enzyme that modulates acetylation, with the *proviso* that (a) the enzyme that modulates acetylation is not a sirtuin protein, (b) the enzyme that modulates acetylation is not human SIRT1, (c) the enzyme that modulates acetylation is not a SIRT1 protein, (d) the enzyme that modulates acetylation is not human SIRT3, (e) the enzyme that modulates acetylation is not a SIRT3 protein, (f) the enzyme that modulates acetylation is not a SIRT1 or SIRT3 protein, (g) the peptide substrate is not Ac-EE-K(biotin)-GQSTSSHSK(Ac)NleSTEG-K(MR121)-EB-$NH_2$ (SEQ ID NO: 1), (h) the peptide substrate is not Ac-EE-K(biotin)-GQSTSSHSK(Ac)NleSTEG-K(5TMR)-EE-NH2 (SEQ ID NO: 2), (i) the peptide substrate is not Biotin-GASSHSK(Ac)VLK(MR121) (SEQ ID NO: 23), (j) the peptide substrate is not GQSTSSHSK(Ac) NleSTEG (SEQ ID NO: 36) wherein the peptide substrate is labeled with a fluorophore and biotin, (k) the peptide substrate is not GASSHSK(Ac)VLK (SEQ ID NO: 37) wherein the peptide substrte is labeled with a fluorophore and biotin, and/or (l) the peptide substrate is not EEKGQSTSSHSK(Ac)NIeSTEGKEE (SEQ ID NO: 38) wherein the peptide substrate is labeled with a fluorophore and biotin.

## EXEMPLIFICATION

The invention now being generally described, it will be more readily understood by reference to the following examples which are included merely for purposes of illustration of certain aspects and embodiments of the present invention

### *EXAMPLE 1: Identification of Sirtuin Modulators Using SIRT1*

A fluorescence polarization based assay was used to identify modulators of SIRT1 activity. The same assay may be used to identify modulators of any sirtuin protein. The fluorescence polarization assays utilizes one of two different peptides based on a fragment of p53, a known sirtuin deacetylation target. Compounds were tested using a substrate containing peptide 1 having 20 amino acid residues as follows: Ac-EE-K(biotin)-GQSTSSHSK(Ac)NleSTEG-K (MR121)-EE-$NH_2$ (SEQ ID NO: 1) wherein K(biotin) is a biotinylated lysine residue, K(Ac) is an acetylated lysine residue, Nle is norleucine and K(MR121) is a lysine residue modified by an MR121 fluorophore. This peptide is labeled with the fluorophore MR121 (excitation 635 nm/emission 680 nm) at the C-termini and biotin at the N-termini. The sequence of the peptide substrates are based on p53 with several modifications. In particular, all arginine and leucine residues other than the acetylated lysine residues have replaced with serine so that the peptides are not susceptible to trypsin cleavage in the absence of deacetylation. In addition, the methionine residues naturally present in the sequences have been replaced with the norleucine because the methionine may be susceptible to oxidation during synthesis and purification. As an alternative substrate in the assay, the following peptide 2 has also been used: Ac-EE-K(biotin)-GQSTSSHSK(Ac) NleSTEG-K(5TMR)-EE-NH2 (SEQ ID NO: 2) wherein K(Ac) is an acetylated lysine residue and Nle is a norleucine. The

peptide is labeled with the fluorophore 5TMR (excitation 540 nm/emission 580 nm) at the C-terminus. The sequence of the peptide substrate is also based on p53 with several modifications. In addition, the methionine residue naturally present in the sequence was replaced with the norleucine because the methionine may be susceptible to oxidation during synthesis and purification.

The peptide substrates were exposed to a sirtuin protein in the presence of $NAD^+$ to allow deacetylation of the substrate and render it sensitive to cleavage by trypsin. Trypsin was then added and the reaction was carried to completion (i.e., the deacetylated substrate is cleaved) releasing the MR121 or 5TMR fragment. Streptavidin is then added to the reaction where it can bind both the uncleaved substrate (i.e., any remaining acetylated substrate) and the non-fluorescent portion of the cleaved peptide substrate (i.e., the biotin containing fragment). The fluorescence polarization signal observed for the full length peptide substrates bound to streptavidin was higher than the fluorescence polarization signal observed for the released MR121 or 5TMR C-terminal fragment. In this way, the fluorescence polarization obtained is inversely proportional to the level of deacetylation (e.g., the signal is inversely proportional to the activity of the sirtuin protein). Results were read on a microplate fluorescence polarization reader (Molecular Devices Spectramax MD) with appropriate excitation and emission filters.

The fluorescence polarization assays using peptide 1 is conducted as follows: 0.5 μM peptide substrate and 150 μM $\beta NAD^+$ is incubated with 0.1 μg/mL of SIRT1 for 60 minutes at 37°C in a reaction buffer (25 mM Tris-acetate pH8, 137 mM Na-Ac, 2.7 mM K-Ac, 1 mM Mg-Ac, 0.05% Tween-20, 0.1% Pluronic F127, 10 mM $CaCl_2$, 5 mM DTT, 0.025% BSA, 0.15 mM Nicotinamide). Test compounds 1-18 were solubilized in DMSO and added to the reaction at 11 concentrations ranging from 0.7 μM to 100 μM.

Fluorescence polarization assays using peptide 2 is conducted as follows: 0.5 μM peptide substrate and 120 μM $\beta NAD^+$ were incubated with 3 nM SIRT1 for 20 minutes at 25°C in a reaction buffer (25 mM Tris-acetate pH8, 137 mM Na-Ac, 2.7 mM K-Ac, 1 mM Mg-Ac, 0.05% Tween-20, 0.1% Pluronic F127, 10 mM $CaCl_2$, 5 mM DTT, 0.025% BSA). Test compounds 19-56 were solubilized in DMSO and added to the reaction at 10 concentrations ranging from 300 μM to 0.15 μM in three-fold dilutions.

After the incubation with SIRT1, nicotinamide was added to the reaction to a final concentration of 3 mM to stop the deacetylation reaction and 0.5 μg/mL of trypsin was added to cleave the deacetylated substrate. The reaction was incubated for 30 minutes at 37°C in the presence of 1 μM streptavidin. Fluorescent polarization was determined at excitation (650 nm) and emission (680 nm) wavelengths. The level of activity of the sirtuin protein in the presence of the various concentrations of test compound is then determined and may be compared to the level of activity of the sirtuin protein in the absence of the test compound, and/or the level of activity of the sirtuin proteins in the negative control (e.g., level of inhibition) and positive control (e.g., level of activation) described below.

For the Fluorescence Polarization assays, a control for inhibition of sirtuin activity is conducted by adding 1 μL of 500 mM nicotinamide as a negative control at the start of the reaction (e.g., permits determination of maximum sirtuin inhibition). A control for activation of sirtuin activity was conducted using 3 nM of sirtuin protein, with 1 μL of DMSO in place of compound, to reach baseline deacetylation of the substrate (e.g., to determine normalized sirtuin activity).

For each of the above assays, SIRT1 protein was expressed and purified as follows. The SirT1 gene was cloned into a T7-promoter containing vector and transformed into BL21 (DE3). The protein was expressed by induction with 1 mM IPTG as an N-terminal His-tag fusion protein at 18°C overnight and harvested at 30,000 x g. Cells were lysed with lysozyme in lysis buffer (50 mM Tris-HCl, 2 mM Tris[2-carboxyethyl] phosphine (TCEP), 10 μM $ZnCl_2$, 200 mM NaCl) and further treated with sonication for 10 min for complete lysis. The protein was purified over a Ni-NTA column (Amersham) and fractions containing pure protein were pooled, concentrated and run over a sizing column (Sephadex S200 26/60 global). The peak containing soluble protein was collected and run on an Ion-exchange column (MonoQ). Gradient elution (200 mM - 500 mM NaCl) yielded pure protein. This protein was concentrated and dialyzed against dialysis buffer (20 mM Tris-HCl, 2 mM TCEP) overnight. The protein was aliquoted and frozen at -80°C until further use.

Sirtuin modulating compounds that activated SIRT1 were identified using the assay described above and are shown below in Table 4. Sirtuin modulating compounds that inhibited SIRT1 were identified using the assay described above and are shown below in Table 5. The $ED_{50}$ values for the activating compounds are represented by A ($ED_{50}$ = <50 μM), B ($ED_{50}$ = 51-100 μM), C ($ED_{50}$ =101-150 μM), and D ($ED_{50}$ = >150 μM). NT denotes compounds that were not tested, often times due to solubility issues. The -$ED_{50}$ of resveratrol for activation of SIRT1 is 16 μM. Similarly, the $IC_{50}$ values for the inhibiting compounds are represented by A ($IC_{50}$ = <50 μM), B ($IC_{50}$ = 51-100 μM), C ($IC_{50}$ =101-150 μM), and D ($IC_{50}$ = >150 μM).

Table 4.

| COMPOUND No | STRUCTURE | $ED_{50}$ |
|---|---|---|
| 1 | 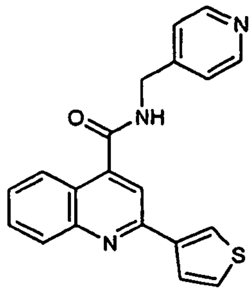 | A |
| 2 | 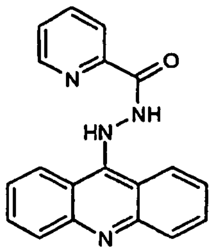 | B |
| 3 | 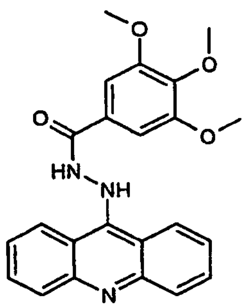 | A |
| 4 | 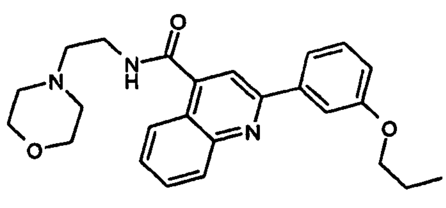 | D |
| 5 | 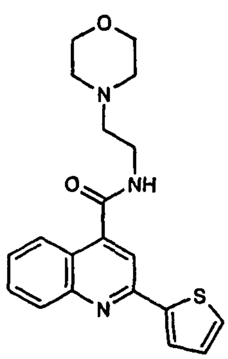 | C |

(continued)

| COMPOUND No | STRUCTURE | $ED_{50}$ |
|---|---|---|
| 6 | 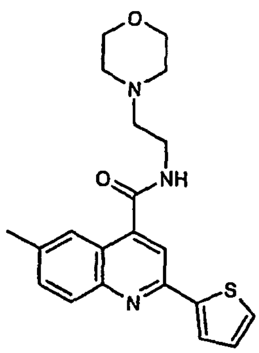 | A |
| 7 | 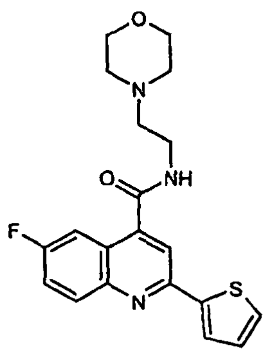 | C |
| 8 | 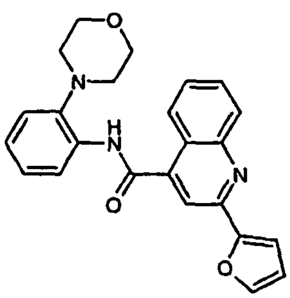 | A |
| 9 | 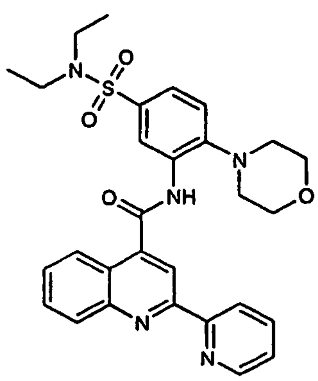 | D |

(continued)

| COMPOUND No | STRUCTURE | ED50 |
|---|---|---|
| 10 | 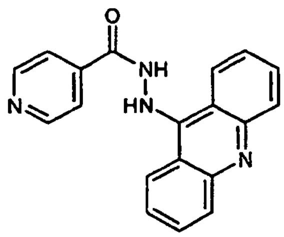 | A |
| 11 | 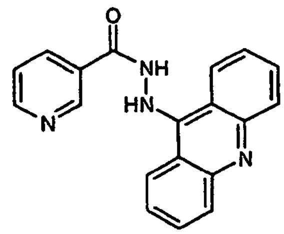 | A |
| 12 | 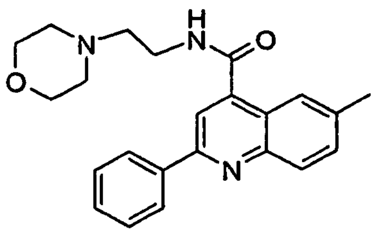 | B |
| 13 | 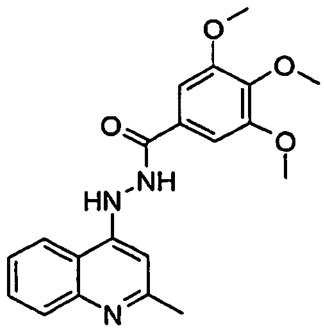 | D |
| | | |
| 14 | 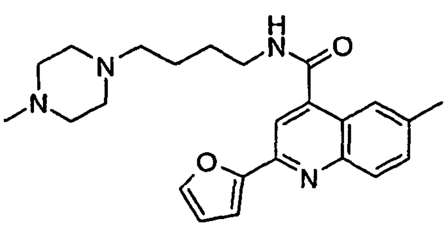 | D |

(continued)

| COMPOUND No | STRUCTURE | $ED_{50}$ |
|---|---|---|
| 15 | 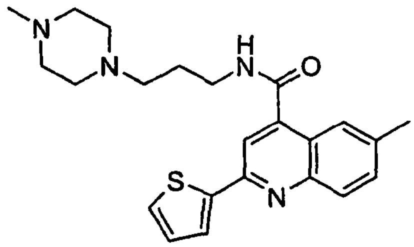 | D |
| 16 | 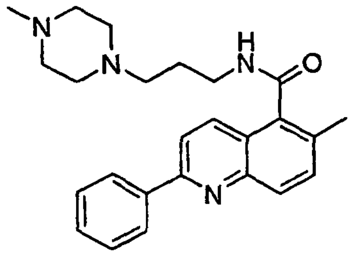 | D |
| 17 | 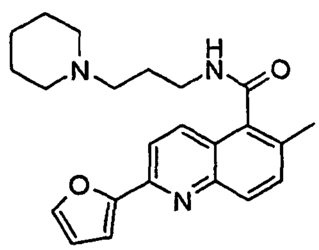 | D |
| 18 | 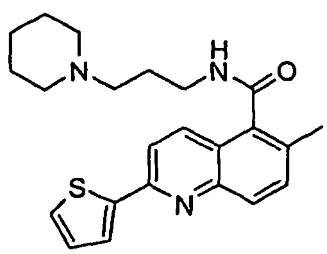 | D |
| 19 | 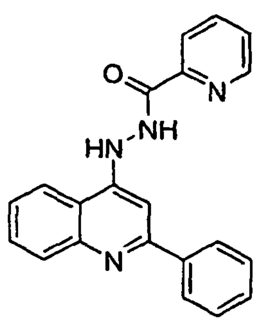 | A |

(continued)

| COMPOUND No | STRUCTURE | $ED_{50}$ |
|---|---|---|
| 20 | 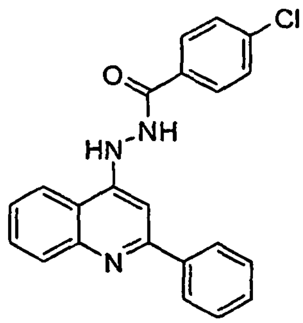 | A |
| 21 | 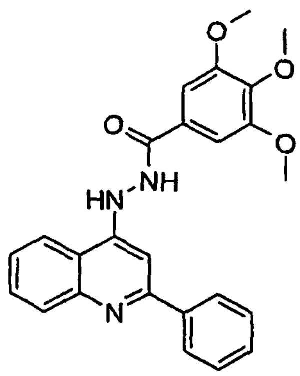 | D |
| 22 | 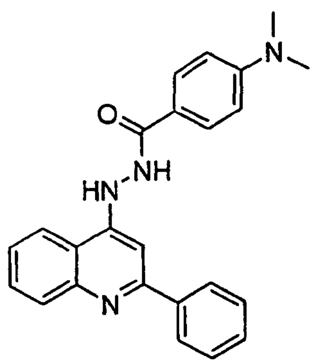 | D |
| 23 | 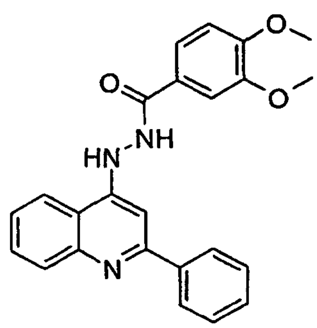 | A |

(continued)

| COMPOUND No | STRUCTURE | $ED_{50}$ |
|---|---|---|
| 24 | 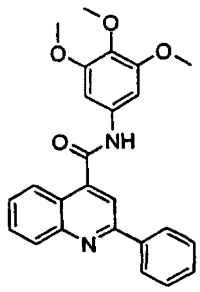 | D |
| 25 | 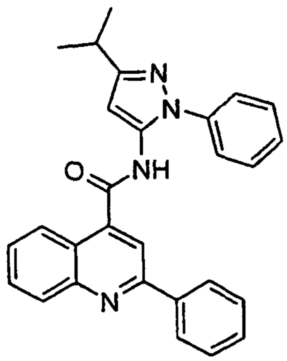 | D |
| 26 | 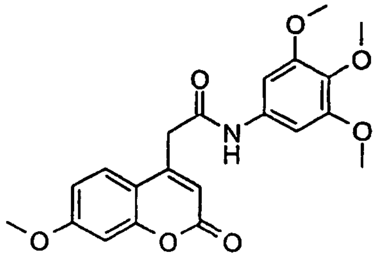 | D |
| 27 | 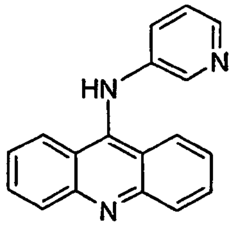 | NT |
| 28 | 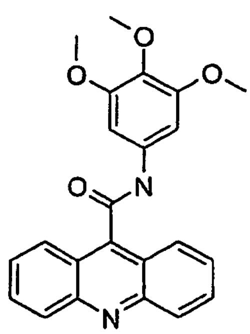 | NT |

(continued)

| COMPOUND No | STRUCTURE | $ED_{50}$ |
|---|---|---|
| 29 | 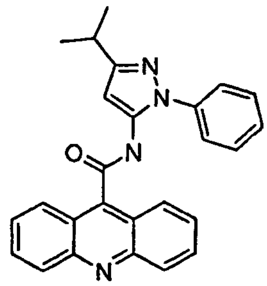 | A |
| 30 | 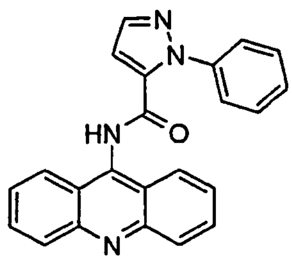 | NT |
| 31 | 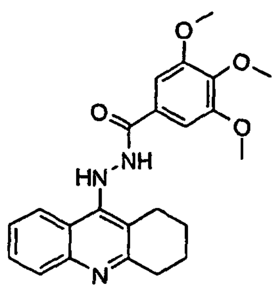 | A |
| 32 | 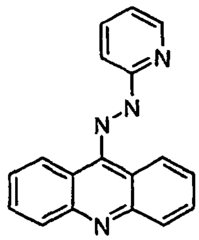 | A |
| 33 | 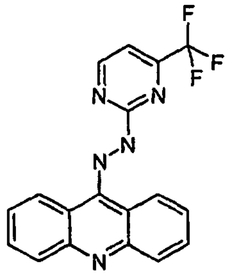 | C |

(continued)

| COMPOUND No | STRUCTURE | $ED_{50}$ |
|---|---|---|
| 34 | 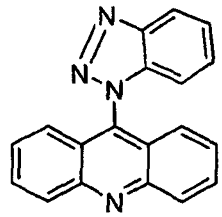 | NT |
| 35 | 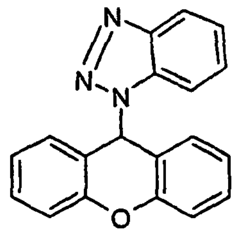 | D |
| 36 | 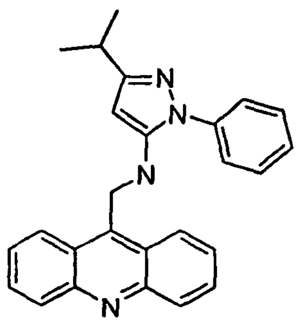 | D |
| 37 | 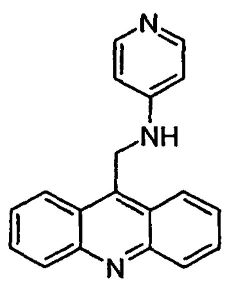 | NT |
| 38 | 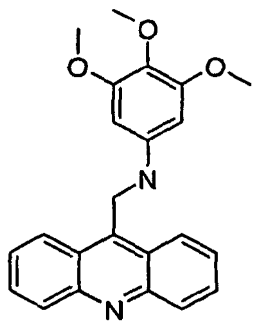 | D |

(continued)

| COMPOUND No | STRUCTURE | $ED_{50}$ |
|---|---|---|
| 39 | 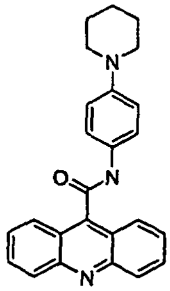 | D |
| 40 | 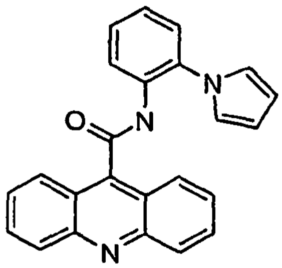 | D |
| 41 | 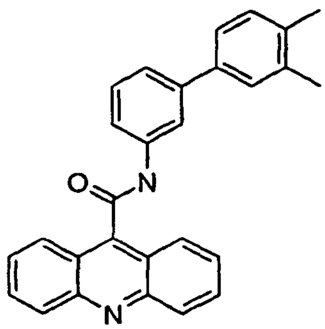 | D |
| 42 | 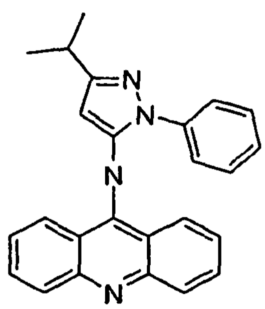 | D |
| 43 | 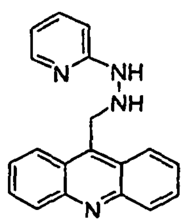 | A |

(continued)

| COMPOUND No | STRUCTURE | $ED_{50}$ |
|---|---|---|
| 44 | 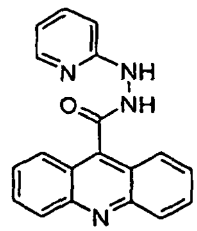 | A |
| 45 | 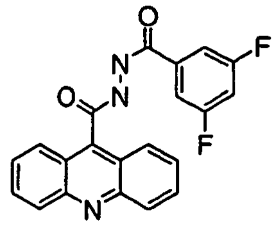 | D |
| 46 | 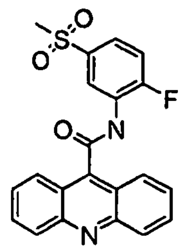 | D |
| 47 | 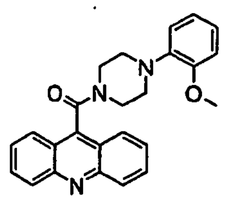 | D |
| 48 | 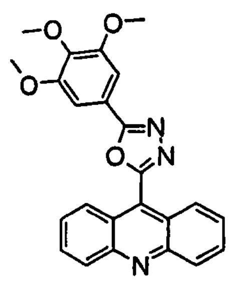 | A |

(continued)

| COMPOUND No | STRUCTURE | $ED_{50}$ |
|---|---|---|
| 49 | 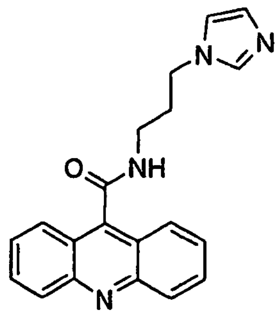 | NT |
| 50 | 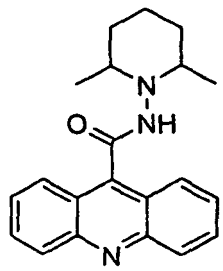 | NT |
| 51 | 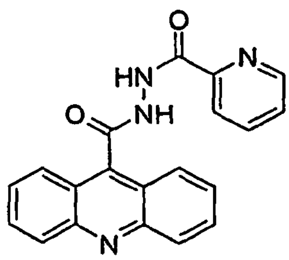 | NT |
| 52 | 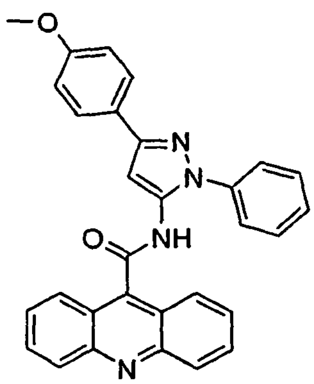 | NT |

(continued)

| COMPOUND No | STRUCTURE | $ED_{50}$ |
|---|---|---|
| 53 | 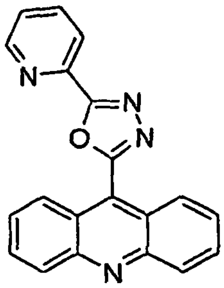 | NT |
| 54 | 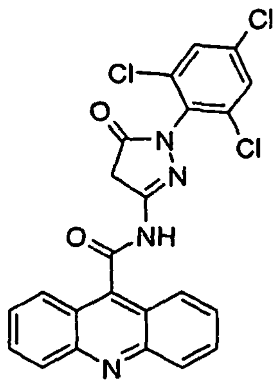 | NT |
| 55 | 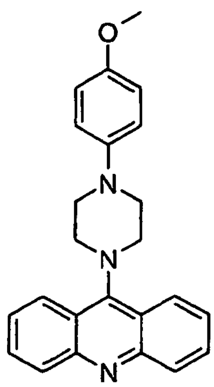 | NT |
| 56 | 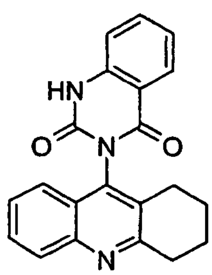 | NT |

(continued)

| COMPOUND No | STRUCTURE | $ED_{50}$ |
|---|---|---|
| 57 | 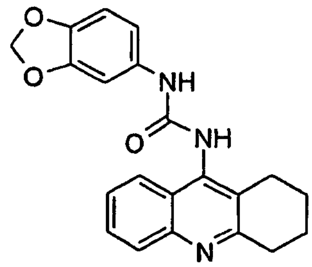 | NT |
| 58 | 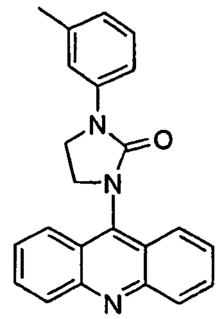 | NT |
| 59 | 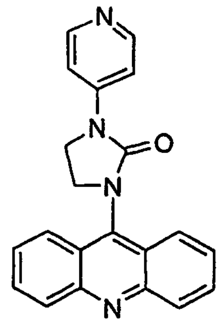 | NT |
| 60 | 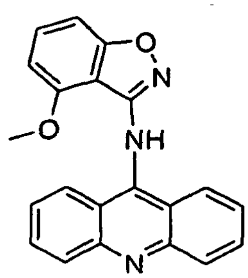 | NT |
| 61 | 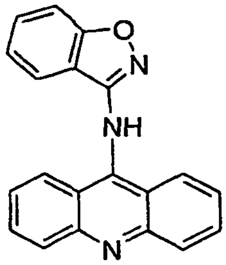 | NT |

(continued)

| COMPOUND No | STRUCTURE | ED$_{50}$ |
|---|---|---|
| 62 | 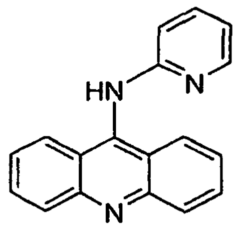 | NT |
| 63 | 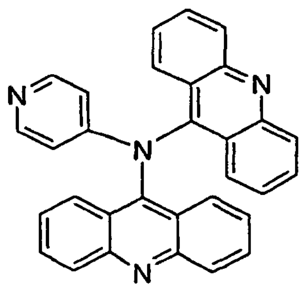 | NT |
| 64 | 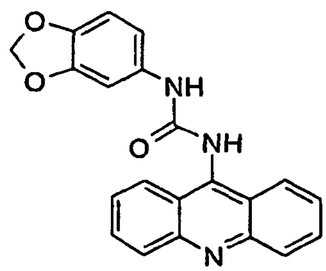 | D |
| 65 | 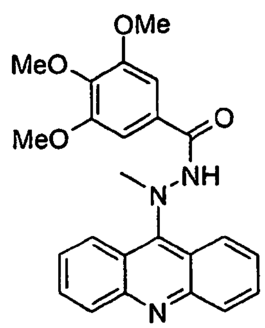 | D |
| 66 | 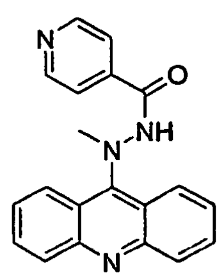 | A |

(continued)

| COMPOUND No | STRUCTURE | $ED_{50}$ |
|---|---|---|
| 67 | 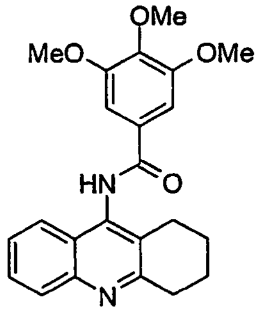 | NT |
| 68 | 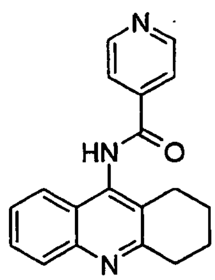 | D |
| 69 | 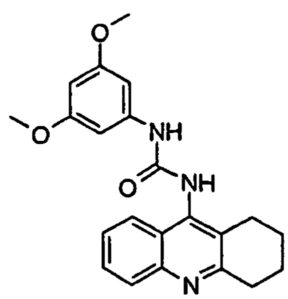 | NT |
| 70 | 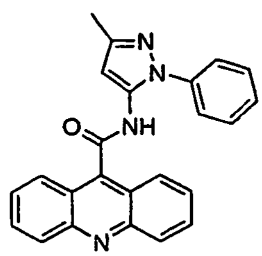 | NT |
| 71 | 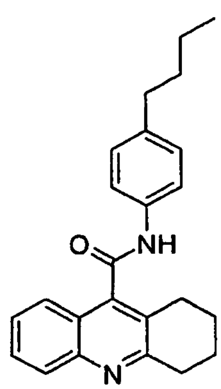 | D |

Table 5.

| COMPOUND No | STRUCTURE | $IC_{50}$ |
|---|---|---|
| 72 | 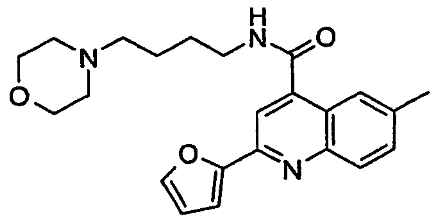 | D |
| 73 | 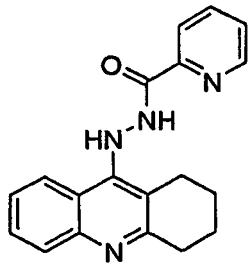 | A |
| 74 | 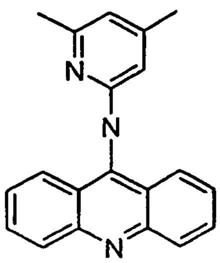 | D |
| 75 | 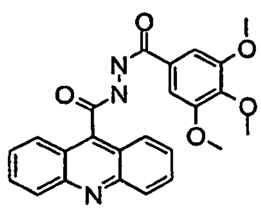 | A |
| 76 | 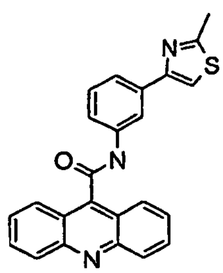 | D |

(continued)

| COMPOUND No | STRUCTURE | $IC_{50}$ |
|---|---|---|
| 77 | 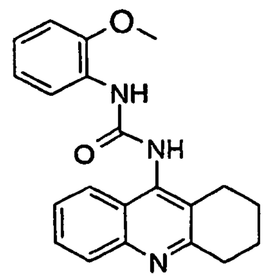 | D |
| 78 | 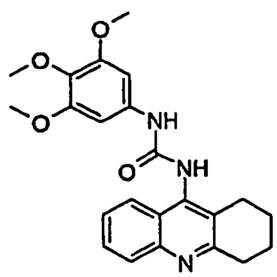 | C |
| 79 | 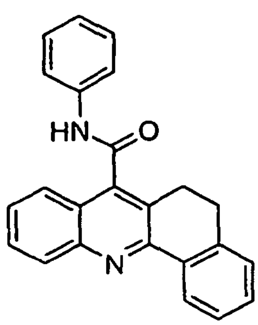 | D |

***EXAMPLE 2: Identification of Sirtuin Modulators Using SIRT2***

A fluorescence polarization assay was used to identify modulators of SIRT2 activity. The same assay may be used to identify modulators of any sirtuin protein. The assay utilizes a peptide substrate based on a fragment of alpha-tubulin, a known sirtuin deacetylation target. The substrate contains a peptide having 14 amino acid residues as follows: Biotin-Nle-PSD-K(Ac)-TIGG-K(NIR121)-NH2 (SEQ ID NO: 9) wherein K(Ac) is an acetylated lysine residue. The peptide is labeled with the fluorophore MR121 (excitation 635 nm/emission 680 nm) at the C-terminus and biotin at the N-terminus.

The peptide substrate is exposed to a sirtuin protein in the presence of $NAD^+$ to allow deacetylation of the substrate and render it sensitive to cleavage by trypsin. Trypsin is then added and the reaction is carried to completion (i.e., the deacetylated substrate is cleaved) releasing the MR121 fragment. Streptavidin is then added to the reaction where it can bind both the uncleaved substrate (i.e., any remaining acetylated substrate) and the non-fluorescent portion of the cleaved peptide substrate (i.e., the biotin containing fragment). The fluorescence polarization signal observed for the full length peptide substrate bound to streptavidin is higher than the fluorescence polarization signal observed for the released MR121 C-terminal fragment. Therefore, the fluorescence polarization obtained is inversely proportional to the level of deacetylation (e.g., the signal is inversely proportional to the activity of the sirtuin protein). Results are read on a microplate fluorescence polarization reader (Molecular Devices Spectramax MD) with appropriate excitation and emission filters.

The fluorescence polarization assays may be conducted as follows: 0.5 $\mu$M peptide substrate and 35.7 $\mu$M $\beta NAD^+$ is incubated with 215nM of SIRT2 for 60 minutes at 37˚C in a reaction buffer (25 mM Tris-acetate pH8, 137 mM Na-Ac, 2.7 mM K-Ac, 1 mM Mg-Ac, 0.1% Pluronic F127, 10 mM $CaCl_2$, 1 mM TCEP, 0.025% BSA). Test compounds are solubilized in DMSO and are added to the reaction at 11 concentrations ranging from 0.7 $\mu$M to 100 $\mu$M. Human SIRT2 enzyme used in the assay was purchased (Biomol International, Cat. No. SE-251, Plymouth Meeting, PA). As

an alternative source, the SIRT2 protein used in the assays corresponded to amino acid residues 1-319 of human SIRT2 with an N-terminal His-tag can be used. The protein is overexpressed in E. coli and purified on a nickel chelate column using standard techniques. After the 60 minute incubation with SIRT2, nicotinamide is added to the reaction to a final concentration of 3 mM to stop the deacetylation reaction and 0.5 μg/mL of trypsin is added to cleave the deacetylated substrate. The reaction is incubated for 30 minutes at 37˚C in the presence of 1 mM streptavidin. Fluorescent polarization is determined at excitation (650 nm) and emissions (680 nm) wavelengths. The level of activity of the sirtuin protein in the presence of the various concentrations of test compound are then determined and may be compared to the level of activity of the sirtuin protein in the absence of the test compound, and/or the level of activity of the sirtuin proteins in the negative control (e.g., level of inhibition) and positive control (e.g., level of activation) described below.

A control for inhibition of sirtuin activity is conducted by adding 30 mM nicotinamide at the start of the reaction (e.g., permits determination of maximum sirtuin inhibition). A control for activation of sirtuin activity is conducted using 0.5 μg/mL of sirtuin protein to reach baseline deacetylation of the substrate (e.g., to determine normalized sirtuin activity).

***EXAMPLE 3: Identification of Sirtuin Modulators Using SIRT3***

A fluorescence polarization assay was used to identify modulators of SIRT3 activity. The same assay may be used to identify modulators of any sirtuin protein. The assay utilizes a peptide substrate based on a fragment of Histone H4, a known sirtuin deacetylation target. The substrate contains a peptide having 14 amino acid residues as follows: Biotin-GASSHSK(Ac)VLK(MR121) (SEQ ID NO: 23) wherein K(Ac) is an acetylated lysine residue. The peptide is labeled with the fluorophore MR121 (excitation 635 nm/emission 680 nm) at the C-terminus and biotin at the N-terminus.

The peptide substrate is exposed to a sirtuin protein in the presence of $NAD^+$ to allow deacetylation of the substrate and render it sensitive to cleavage by trypsin. Trypsin is then added and the reaction is carried to completion (i.e., the deacetylated substrate is cleaved) releasing the MR121 fragment. Streptavidin is then added to the reaction where it can bind both the uncleaved substrate (i.e., any remaining acetylated substrate) and the non-fluorescent portion of the cleaved peptide substrate (i.e., the biotin containing fragment). The fluorescence polarization signal observed for the full length peptide substrate bound to streptavidin is higher than the fluorescence polarization signal observed for the released MR121 C-terminal fragment. Therefore, the fluorescence polarization obtained is inversely proportional to the level of deacetylation (e.g., the signal is inversely proportional to the activity of the sirtuin protein). Results are read on a microplate fluorescence polarization reader (Molecular Devices Spectramax MD) with appropriate excitation and emission filters.

The fluorescence polarization assays may be conducted as follows: 0.5 μM peptide substrate and 50 μM $\beta NAD^+$ is incubated with 2 nM of SIRT3 for 60 minutes at 37˚C in a reaction buffer (25 mM Tris-acetate pH8, 137 mM Na-Ac, 2.7 mM K-Ac, 1 mM Mg-Ac, 0.1% Pluronic F127, 10 mM $CaCl_2$, 1 mM TCEP, 0.025% BSA). Test compounds are solubilized in DMSO and are added to the reaction at 11 concentrations ranging from 0.7 μM to 100 μM. The SIRT3 protein used in the assays corresponded to amino acid residues 102-399 of human SIRT3 with an N-terminal His-tag. The protein was overexpressed in E. coli and purified on a nickel chelate column using standard techniques. After the 60 minute incubation with SIRT3, nicotinamide is added to the reaction to a final concentration of 3 mM to stop the deacetylation reaction and 0.5 μg/mL of trypsin is added to cleave the deacetylated substrate. The reaction is incubated for 30 minutes at 37˚C in the presence of 1 mM streptavidin. Fluorescent polarization is determined at excitation (650 nm) and emissions (680 nm) wavelengths. The level of activity of the sirtuin protein in the presence of the various concentrations of test compound are then determined and may be compared to the level of activity of the sirtuin protein in the absence of the test compound, and/or the level of activity of the sirtuin proteins in the negative control (e.g., level of inhibition) and positive control (e.g., level of activation) described below.

A control for inhibition of sirtuin activity is conducted by adding 30 mM nicotinamide at the start of the reaction (e.g., permits determination of maximum sirtuin inhibition). A control for activation of sirtuin activity is conducted using 0.5 μg/mL of sirtuin protein to reach baseline deacetylation of the substrate (e.g., to determine normalized sirtuin activity).

Sirtuin modulating compounds that activated SIRT3 were identified using the assay described above and are shown below in Table 6. Sirtuin modulating compounds that inhibited SIRT3 were identified using the assay described above and are shown below in Table 7. The $ED_{50}$ values for the activating compounds are represented by A ($ED_{50}$ = <50 μM), B ($ED_{50}$ = 51-100 μM), C ($ED_{50}$ =101-150 μM), and D ($ED_{50}$ = >150 μM). The $ED_{50}$ of resveratrol for activation of SIRT3 is >300 μM. Similarly, the $IC_{50}$ values for the inhibiting compounds are represented by A ($IC_{50}$ = <50 μM), B ($IC_{50}$ = 51-100 μM), C ($IC_{50}$ = 101-150 μM), and D ($IC_{50}$ = >150 μM).

Table 6.

| COMPOUND No | STRUCTURE | $ED_{50}$ |
|---|---|---|
| 1 | 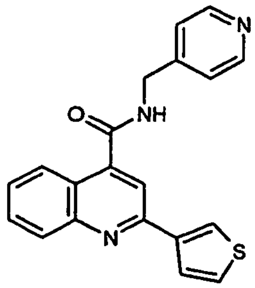 | B |
| 8 | 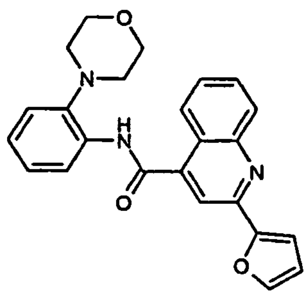 | A |
| 11 | 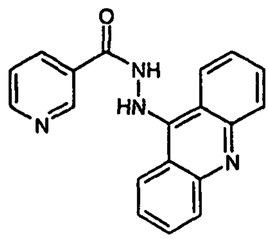 | B |
| 80 | 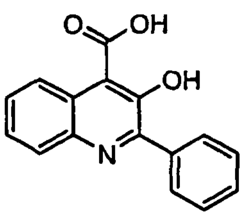 | B |

Table 7.

| | | |
|---|---|---|
| 81 | 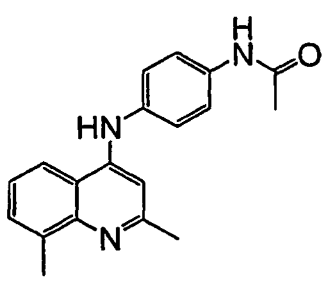 | C |

(continued)

| | | |
|---|---|---|
| 82 | 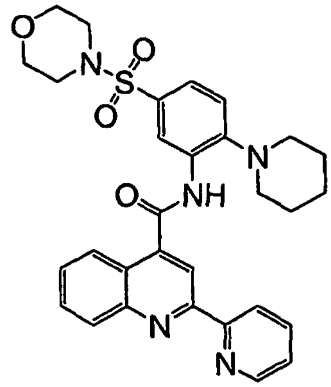 | N/A |

## Claims

1. A method for determining the activity of a deacetylase, comprising:

   contacting a peptide substrate pool with a deacetylase, wherein members of said peptide substrate pool comprise a fluorophore and at least one acetylated lysine residue;
   contacting the peptide substrate pool with a reagent that cleaves members of the peptide substrate pool having non-acetylated lysine residues; and
   determining the fluorescence polarization value of the peptide substrate pool, wherein a decrease in the fluorescence polarization value of the peptide substrate pool is indicative of deacetylase activity.

2. A method for identifying a compound that modulates a deacetylase, comprising:

   contacting a peptide substrate pool with a deacetylase in the presence of a test compound, wherein members of said peptide substrate pool comprise a fluorophore and at least one acetylated lysine residue;
   contacting the peptide substrate pool with a reagent that cleaves members of the peptide substrate pool having non-acetylated lysine residues; and
   determining the fluorescence polarization value of the peptide substrate pool, wherein a change in the fluorescence polarization value of the peptide substrate pool in the presence of the test compound as compared to a control is indicative of a compound that modulates the activity of the deacetylase.

3. The method of claim 1 or 2, wherein the members of said peptide substrate pool further comprise a bulky group and said at least one acetylated lysine residue is located between the fluorophore and the bulky group.

4. The method of claim 3, wherein said bulky group is biotin, a blotin-avidin complex, or a biotin-streptavidin complex.

5. The method of claim 1 or 2, wherein the members of said peptide substrate pool further comprises a binding site and said at least one acetylated lysine residue is located between the fluorophore and the binding site.

6. A method for determining the activity of an acetyltransferase, comprising:

   contacting a peptide substrate pool with an acetyltransferase, wherein members of said peptide substrate pool comprise a fluorophore and at feast one **lysine** residue;
   contacting the peptide substrate pool with a reagent that cleaves members of the peptide substrate pool having non-acetylated lysine residues; and
   determining the fluorescence polarization value of the peptide substrate pool, wherein an increase in the fluorescence polarization value of the peptide substrate pool is indicative of acetyltransferase activity.

7. A method for identifying a compound that modulates an acetyltransferase, comprising:

   contacting a peptide substrate pool with an acetyltransferase in the presence of a test compound, wherein

members of said peptide substrate pool comprise a fluorophore and at least one lysine residue located between the fluorophore and the binding site;
contacting the peptide substrate pool with a reagent that cleaves molecules of the peptide substrate having non-acetylated lysine residues; and
determining the Fluorescence polarization value of the peptide substrate pool, wherein a change in the fluorescence polarization value of the peptide substrate pool In the presence of the test compound as compared to a control is indicative of a compound that modulates the activity of the acetyltransferase.

**8.** The method of claim 8 or 7, wherein the members of said peptide substrate pool further comprise a bulky group and said at least one lysine residue is located between the fluorophore and the bulky group.

**9.** The method of claim 8, wherein said bulky group is biotin, a biotin-avidin complex, or a biotin-streptavidin complex.

**10.** The method of claim 6 or 7, wherein the members of said peptide substrate pool further comprises a binding site and said at least one lysine residue is located between the fluorophore and the binding site.

**11.** The method of claim 5 or 10, wherein said method further comprises contacting the peptide substrate pool with a binding moiety that binds to said binding site.

**12.** The method of claim 11. wherein the binding site is an antigen.

**13.** The method of claim 12, wherein the binding moiety is an antibody.

**14.** The method of claim 11, wherein the binding site is biotin.

**15.** The method of claim 14, wherein the binding moiety is avidin or streptavidin.

**16.** The method of claim 11, wherein the binding site comprises an Fc region, or a portion thereof.

**17.** The method of claim 16, wherein the binding moiety is protein A or protein G.

**18.** The method of claim 1 or 2, wherein the deacetylase is a histone deacetylase (HDAC) or a sirtuin.

**19.** The method of claim 18, wherein the sirtuin is a SIRT1 protein.

**20.** The method of claim 6 or 7, wherein the acetyltransferase is Gcn5 or p300/CBP.

**21.** The method of claim 1, 2, 6 or 7, wherein the reagent that cleaves the peptide substrate is a protease.

**22.** The method of claim 21, wherein the protease is lysylendopeptidase, endoproteinase, Lys-C, plasmin, calpain, or trypsin.

**23.** The method of claim 1, 2, 6 or 7, wherein the fluorophore is MR121 or 5TMR.

**24.** The method of claim 1, 2, 6 or 7, wherein the sequence of the peptide substrate is derived from a histone, an HMG protein, p53, c-Myb, GATA-1, EKLF, MyoD, E2F, dTCF, or HIV Tat, or a fragment thereof.

**25.** The method of claim 2 or 7, wherein the compound is a small molecule.

**26.** The method of claim 2, wherein a compound that increases the activity of the deacetylase is Identified.

**27.** The method of claim 28, wherein a decrease in the fluorescence polarization value of the peptide substrate pool in the presence of the test compound as compared to a control is indicative of a compound that increases the activity of the deacetylase.

**28.** The method of claim 2, wherein a compound that inhibits the activity of the deacetylase is identified.

**29.** The method of claim 28, wherein an increase in the fluorescence polarization value of the peptide substrate pool

in the presence of the test compound as compared to a control is Indicative of a compound that inhibits the activity of the deacetylase.

**30.** The method of claim 7, wherein a compound that inhibite the activity of the acetyltransferase Is identified.

**31.** The method of claim 30, wherein a decrease in the fluorescence polarization value of the peptide substrate pool upon contact with the acetyltraneferase in the presence of the test compound as compared to a control is indicative of a compound that inhibits the activity of the acetyltransferase.

**32.** The method of claim 7, wherein a compound that increases the activity of the acetyltransferese is identified.

**33.** The method of claim 32, wherein an increase In the fluorescence polarization value of the peptide substrate pool upon contact with the acetyltransferase in the presence of the test compound as compared to a control is indicative of a compound that increases the activity of the acetyltransferase.

**34.** A method for determining the activity of an enzyme that modulates acetylation of a peptide substrate, comprising:

contacting a peptide substrate pool with an enzyme that modulates acetylation, wherein members of said peptide substrate pool comprise a fluorophore and at least one lysine residue or acetylated lysine residue;
contacting the peptide substrate pool with a cleavage reagent that cleaves members of the peptide substrate pool having non-acetylated lysine residues; and
determining the fluorescence polarization value of the peptide substrate pool, wherein a change in the fluorescence polarization value of the peptide substrate pool is indicative of activity of the enzyme that modulates acetylation.

**35.** A method for identifying a compound that modulates the activity of an enzyme that modulates acetylation of a peptide substrate, comprising:

contacting a peptide substrate pool with an enzyme that modulates acetylation in the presence of a test compound, wherein members of said peptide substrate pool comprise a fluorophore and at least one lysine residue or acetylated lysine residue;
contacting the peptide substrate pool with a cleavage reagent that cleaves members of the peptide substrate pool having non-acetylated lysine residues; and
determining the fluorescence polarization value of the peptide substrate pool, wherein a change in the fluorescence polarization value of the peptide substrate pool in the presence of the test compound is indicative of a compound that modulates the activity of the enzyme that modulates acetylation.

## Patentansprüche

**1.** Verfahren zur Bestimmung der Aktivität einer Deacetylase, wobei das Verfahren aufweist:

Inkontaktbringen eines Peptidsubstrat-Pools mit einer Deacetylase, wobei Elemente des Peptidsubstrat-Pools einen Fluorophor und mindestens einen acetylierten Lysinrest aufweisen;
Inkontaktbringen des Peptidsubstrat-Pools mit einem Reagenz, das Elemente des Peptidsubstrat-Pools mit nicht acetylierten Lysinresten aufspaltet; und
Bestimmen des Fluoreszenzpolarisationswerts des Peptidsubstrat-Pools, wobei eine Abnahme des Fluoreszenzpolarisationswerts des Peptidsubstrat-Pools Deacetylase-Aktivität anzeigt.

**2.** Verfahren zur Identifikation einer Verbindung, die eine Deacetylase moduliert, wobei das Verfahren aufweist:

Inkontaktbringen eines Peptidsubstrat-Pools mit einer Deacetylase in Anwesenheit einer Testverbindung, wobei Elemente des Peptidsubstrat-Pools einen Fluorophor und mindestens einen acetylierten Lysinrest aufweisen;
Inkontaktbringen des Peptidsubstrat-Pools mit einem Reagenz, das Elemente des Peptidsubstrat-Pools mit nicht acetylierten Lysinresten aufspaltet; und
Bestimmen des Fluoreszenzpolarisationswerts des Peptidsubstrat-Pools, wobei eine Veränderung des Fluoreszenzpolarisationswerts des Peptidsubstrat-Pools in Anwesenheit der Testverbindung im Vergleich zu einer Kontrolle eine Verbindung anzeigt, welche die Aktivität der Deacetylase moduliert.

3. Verfahren nach Anspruch 1 oder 2, wobei die Elemente des Peptidsubstrat-Pools ferner eine sperrige Gruppe aufweisen und der mindestens eine acetylierte Lysinrest zwischen dem Fluorophor und der sperrigen Gruppe angeordnet ist.

4. Verfahren nach Anspruch 3, wobei die sperrige Gruppe Biotin, ein Biotin-Avidin-Komplex oder ein Biotin-Streptavidin-Komplex ist.

5. Verfahren nach Anspruch 1 oder 2, wobei die Elemente des Peptidsubstrat-Pools ferner eine Bindungsstelle aufweisen und der mindestens eine acetylierte Lysinrest zwischen dem Fluorophor und der Bindungsstelle angeordnet ist.

6. Verfahren zur Bestimmung der Aktivität einer Acetyltransferase, wobei das Verfahren aufweist:

Inkontaktbringen eines Peptidsubstrat-Pools mit einer Acetyltransferase, wobei Elemente des Peptidsubstrat-Pools einen Fluorophor und mindestens einen Lysinrest aufweisen;
Inkontaktbringen des Peptidsubstrat-Pools mit einem Reagenz, das Elemente des Peptidsubstrat-Pools mit nicht acetylierten Lysinresten aufspaltet; und
Bestimmen des Fluoreszenzpolarisationswerts des Peptidsubstrat-Pools, wobei eine Zunahme des Fluoreszenzpolarisationswerts des Peptidsubstrat-Pools Acetyltransferase-Aktivität anzeigt.

7. Verfahren zur Identifikation einer Verbindung, die eine Acetyltransferase moduliert, wobei das Verfahren aufweist:

Inkontaktbringen eines Peptidsubstrat-Pools mit einer Acetyltransferase in Anwesenheit einer Testverbindung, wobei Elemente des Peptidsubstrat-Pools einen Fluorophor und mindestens einen Lysinrest aufweisen, der zwischen dem Fluorophor und der Bindungsstelle angeordnet ist;
Inkontaktbringen des Peptidsubstrat-Pools mit einem Reagenz, das Moleküle des Peptidsubstrats mit nicht acetylierten Lysinresten aufspaltet; und
Bestimmen des Fluoreszenzpolarisationswerts des Peptidsubstrat-Pools, wobei eine Veränderung des Fluoreszenzpolarisationswerts des Peptidsubstrat-Pools in Anwesenheit der Testverbindung im Vergleich zu einer Kontrolle eine Verbindung anzeigt, welche die Aktivität der Acetyltransferase moduliert.

8. Verfahren nach Anspruch 6 oder 7, wobei die Elemente des Peptidsubstrat-Pools ferner eine sperrige Gruppe aufweisen und der mindestens eine Lysinrest zwischen dem Fluorophor und der sperrigen Gruppe angeordnet ist.

9. Verfahren nach Anspruch 8, wobei die sperrige Gruppe Biotin, ein Biotin-Avidin-Komplex oder ein Biotin-Streptavidin-Komplex ist.

10. Verfahren nach Anspruch 6 oder 7, wobei die Elemente des Peptidsubstrat-Pools ferner eine Bindungsstelle aufweisen und der mindestens eine Lysinrest zwischen dem Fluorophor und der Bindungsstelle angeordnet ist.

11. Verfahren nach Anspruch 5 oder 10, wobei das Verfahren ferner das Inkontaktbringen des Peptidsubstrat-Pools mit einer Bindungskomponente aufweist, die an die Bindungsstelle bindet.

12. Verfahren nach Anspruch 11, wobei die Bindungsstelle ein Antigen ist.

13. Verfahren nach Anspruch 12, wobei die Bindungskomponente ein Antikörper ist.

14. Verfahren nach Anspruch 11, wobei die Bindungsstelle Biotin ist.

15. Verfahren nach Anspruch 14, wobei die Bindungskomponente Avidin oder Streptavidin ist.

16. Verfahren nach Anspruch 11, wobei die Bindungsstelle eine Fc-Region oder einen Teil davon aufweist.

17. Verfahren nach Anspruch 16, wobei die Bindungskomponente Protein A oder Protein G ist.

18. Verfahren nach Anspruch 1 oder 2, wobei die Deacetylase eine Histondeacetylase (HDAC) oder ein Sirtuin ist.

19. Verfahren nach Anspruch 18, wobei das Sirtuin ein SIRTI-Protein ist.

**20.** Verfahren nach Anspruch 6 oder 7, wobei die Acetyltransferase Gcn5 oder p300/CBP ist.

**21.** Verfahren nach Anspruch 1, 2, 6 oder 7, wobei das Reagenz, welches das Peptidsubstrat spaltet, eine Protease ist.

**22.** Verfahren nach Anspruch 21, wobei die Protease Lysylendopeptidase, Endoproteinase, Lys-C, Plasmin, Calpain oder Trypsin ist.

**23.** Verfahren nach Anspruch 1, 2, 6 oder 7, wobei der Fluorophor MR121 oder 5TMR ist.

**24.** Verfahren nach Anspruch 1, 2, 6 oder 7, wobei die Sequenz des Peptidsubstrats von einem Histon, einem HMG-Protein, p53, cMyb, GATA-1, EKLF, MyoD, E2F, dTCF oder HIV Tat oder einem Fragment davon abgeleitet ist.

**25.** Verfahren nach Anspruch 2 oder 7, wobei die Verbindung ein kleines Molekül ist.

**26.** Verfahren nach Anspruch 2, wobei eine Verbindung identifiziert wird, welche die Aktivität der Deacetylase erhöht.

**27.** Verfahren nach Anspruch 26, wobei eine Abnahme des Fluoreszenzpolarisationswerts des Peptidsubstrat-Pools in Anwesenheit der Testverbindung im Vergleich zu einer Kontrolle eine Verbindung anzeigt, welche die Aktivität der Deacetylase erhöht.

**28.** Verfahren nach Anspruch 2, wobei eine Verbindung identifiziert wird, welche die Aktivität der Deacetylase hemmt.

**29.** Verfahren nach Anspruch 28, wobei eine Zunahme des Fluoreszenzpolarisationswerts des Peptidsubstrat-Pools in Anwesenheit der Testverbindung im Vergleich zu einer Kontrolle eine Verbindung anzeigt, welche die Aktivität der Deacetylase hemmt.

**30.** Verfahren nach Anspruch 7, wobei eine Verbindung identifiziert wird, welche die Aktivität der Acetyltransferase hemmt.

**31.** Verfahren nach Anspruch 30, wobei eine Abnahme des Fluoreszenzpolarisationswerts des Peptidsubstrat-Pools bei Kontakt mit der Acetyltransferase in Anwesenheit der Testverbindung im Vergleich zu einer Kontrolle eine Verbindung anzeigt, welche die Aktivität der Acetyltransferase hemmt.

**32.** Verfahren nach Anspruch 7, wobei eine Verbindung identifiziert wird, welche die Aktivität der Acetyltransferase erhöht.

**33.** Verfahren nach Anspruch 32, wobei eine Zunahme des Fluoreszenzpolarisationswerts des Peptidsubstrat-Pools bei Kontakt mit der Acetyltransferase in Anwesenheit der Testverbindung im Vergleich zu einer Kontrolle eine Verbindung anzeigt, welche die Aktivität der Acetyltransferase erhöht.

**34.** Verfahren zur Bestimmung der Aktivität eines Enzyms, das die Acetylierung eines Peptidsubstrats moduliert, wobei das Verfahren aufweist:

Inkontaktbringen eines Peptidsubstrat-Pools mit einem Enzym, das die Acetylierung moduliert, wobei Elemente des Peptidsubstrat-Pools einen Fluorophor und mindestens einen Lysinrest oder acetylierten Lysinrest aufweisen;
Inkontaktbringen des Peptidsubstrat-Pools mit einem Spaltungsreagenz, das Elemente des Peptidsubstrat-Pools mit nicht acetylierten Lysinresten aufspaltet; und
Bestimmen des Fluoreszenzpolarisationswerts des Peptidsubstrat-Pools, wobei eine Veränderung des Fluoreszenzpolarisationswerts des Peptidsubstrat-Pools Aktivität des Enzyms anzeigt, das die Acetylierung moduliert.

**35.** Verfahren zur Identifikation einer Verbindung, welche die Aktivität eines Enzyms moduliert, das die Acetylierung eines Peptidsubstrats moduliert, wobei das Verfahren aufweist:

Inkontaktbringen eines Peptidsubstrat-Pools mit einem Enzym, das die Acetylierung moduliert, in Anwesenheit einer Testverbindung, wobei Elemente des Peptidsubstrat-Pools einen Fluorophor und mindestens einen Lysinrest oder acetylierten Lysinrest aufweisen,

Inkontaktbringen des Peptidsubstrat-Pools mit einem Spaltungsreagenz, das Elemente des Peptidsubstrat-Pools mit nicht acetylierten Lysinresten aufspaltet; und
Bestimmen des Fluoreszenzpolarisationswerts des Peptidsubstrat-Pools, wobei eine Veränderung des Fluoreszenzpolarisationswerts des Peptidsubstrat-Pools in Anwesenheit der Testverbindung eine Verbindung anzeigt, welche die Aktivität des Enzyms moduliert, das die Acetylierung moduliert.

## Revendications

**1.** Méthode de détermination de l'activité d'une désacétylase, comprenant:

mettre un regroupement de substrats peptidiques en contact avec une désacétylase, où les membres dudit regroupement de substrats peptidiques comprennent un fluorophore et au moins un résidu de lysine acétylée;
mettre le regroupement de substrats peptidiques en contact avec un réactif qui clive les membres du regroupement de substrats peptidiques ayant des résidus de lysine non acétylée; et
déterminer la valeur de polarisation de fluorescence du regroupement de substrats peptidiques, où une diminution dans la valeur de polarisation de fluorescence du regroupement de substrats peptidiques est indicative d'une activité de désacétylase.

**2.** Méthode d'identification d'un composé qui module une désacétylase, comprenant:

mettre un regroupement de substrats peptidiques en contact avec une désacétylase en présence d'un composé de test, où les membres dudit regroupement de substrats peptidiques comprennent un fluorophore et au moins un résidu de lysine acétylée;
mettre le regroupement de substrats peptidiques en contact avec un réactif qui clive les membres du regroupement de substrats peptidiques ayant des résidus de lysine non acétylée; et
déterminer la valeur de polarisation de fluorescence du regroupement de substrats peptidiques, où un changement dans la valeur de polarisation de fluorescence du regroupement de substrats peptidiques en présence du composé de test comparée à un témoin, est indicatif d'un composé qui module l'activité de la désacétylase.

**3.** Méthode selon la revendication 1 ou 2, dans laquelle les membres dudit regroupement de substrats peptidiques comprennent en outre un groupe volumineux et ledit au moins un résidu de lysine acétylée est situé entre le fluorophore et le groupe volumineux.

**4.** Méthode selon la revendication 3, dans laquelle ledit groupe volumineux est de la biotine, un complexe de biotine-avidine ou un complexe de biotine-streptavidine.

**5.** Méthode selon la revendication 1 ou 2, dans laquelle les membres dudit regroupement de substrats peptidiques comprennent en outre un site de liaison et ledit au moins un résidu de lysine acétylée est situé entre le fluorophore et le site de liaison.

**6.** Méthode de détermination de l'activité d'une acétyl transférase, comprenant:

mettre un regroupement de substrats peptidiques en contact avec une acétyl transférase, où les membres dudit regroupement de substrats peptidiques comprennent un fluorophore et au moins un résidu de lysine;
mettre le regroupement de substrats peptidiques en contact avec un réactif qui clive les membres du regroupement de substrats peptidiques ayant des résidus de lysine non acétylée; et
déterminer la valeur de polarisation de fluorescence du regroupement de substrats peptidiques, où une augmentation dans la valeur de polarisation de fluorescence du regroupement de substrats peptidiques est indicative d'une activité d'acétyl transférase.

**7.** Méthode d'identification d'un composé qui module une acétyl transférase, comprenant:

mettre un regroupement de substrats peptidiques en contact avec une acétyl transférase en présence d'un composé de test, où les membres dudit regroupement de substrats peptidiques comprennent un fluorophore et au moins un résidu de lysine situé entre le fluorophore et le site de liaison;
mettre le regroupement de substrats peptidiques en contact avec un réactif qui clive les membres du substrat peptidique ayant des résidus de lysine non acétylée; et

déterminer la valeur de polarisation de fluorescence du regroupement de substrats peptidiques, où un changement dans la valeur de polarisation de fluorescence du regroupement de substrats peptidiques en présence du composé de test comparée à un témoin, est indicatif d'un composé qui module l'activité de facétyl transférase.

**8.** Méthode selon la revendication 6 ou 7, dans laquelle les membre dudit regroupement de substrats peptidiques comprennent en outre un groupe volumineux et ledit au moins un résidu de lysine est situé entre le fluorophore et le groupe volumineux.

**9.** Méthode selon la revendication 8, dans laquelle le groupe volumineux est de la biotine, un complexe de biotine-avidine ou un complexe de biotine-streptavidine.

**10.** Méthode selon la revendication 6 ou 7, dans laquelle les membres dudit regroupement de substrats peptidiques comprennent en outre un site de liaison et ledit au moins un résidu de lysine est situé entre le fluorophore et le site de liaison.

**11.** Méthode selon la revendication 5 ou 10, où ladite méthode comprend en outre mettre le regroupement de substrats peptidiques en contact avec une fraction de liaison qui se lie audit site de liaison.

**12.** Méthode selon la revendication 11, dans laquelle le site de liaison est un antigène.

**13.** Méthode selon la revendication 12, dans laquelle le site de liaison est un anticorps.

**14.** Méthode selon la revendication 11, dans laquelle le site de liaison est de la biotine.

**15.** Méthode selon la revendication 14, dans laquelle la fraction de liaison est de l'avidine ou de la streptavidine.

**16.** Méthode selon la revendication 11, dans laquelle le site de liaison comprend une région Fc ou une partie de celle-ci.

**17.** Méthode selon la revendication 16, dans laquelle la fraction de liaison est une protéine A ou une protéine G.

**18.** Méthode selon la revendication 1 ou 2, dans laquelle la désacétylase est une histone désacétylase (HDAC) ou une sirtuine.

**19.** Méthode selon la revendication 18, dans laquelle la sirtuine est une protéine SIRT1.

**20.** Méthode selon la revendication 6 ou 7, dans laquelle l'acétyl transférase est une Gen5 ou un p300/CBP.

**21.** Méthode selon la revendication 1, 2, 6 ou 7, dans laquelle le réactif qui clive le substrat peptidique est une protéase.

**22.** Méthode selon la revendication 21, dans laquelle la protéase est une lysylendopeptidase, une endoprotéinase, une Lys-C, une plasmine, une calpaïne ou une trypsine.

**23.** Méthode selon la revendication 1, 2, 6 ou 7, dans laquelle le fluorophore est MR121 ou 5TMR.

**24.** Méthode selon la revendication 1, 2, 6 ou 7, dans laquelle la séquence du substrat peptidique est dérivée d'une histone, d'une protéine HMG, de p53, c-Myb, GATA-1, EKLF, MyoD, E2F, dTCF ou HIV Tat ou d'un fragement de ceux-ci.

**25.** Méthode selon la revendication 2 ou 7, dans laquelle le composé est une petite molécule.

**26.** Méthode selon la revendication 2, dans laquelle un composé qui augmente l'activité de la désacétylase est identifié.

**27.** Méthode selon la revendication 26, dans laquelle une diminution dans la valeur de polarisation de fluorescence du regroupement de substrats peptidiques en présence du composé de test comparée à un témoin, est indicative d'un composé qui augmente l'activité de la désacétylase.

**28.** Méthode selon la revendication 2, dans laquelle un composé qui inhibe l'activité de la désacétylase est identifié.

**29.** Méthode selon la revendication 28, dans laquelle une augmentation dans la valeur de polarisation de fluororescence du regroupement de substrats peptidiques en présence du composé de test comparée à un témoin, est indicative d'un composé qui inhibe l'activité de la désacétylase.

**30.** Méthode selon la revendication 7, dans laquelle un composé qui inhibe l'activité de l'acétyl transférase est identifié.

**31.** Méthode selon la revendication 30, dans laquelle une diminution dans la valeur de polarisation de fluorescence du regroupement de substrats peptidiques à la mise en contact avec l'acétyl transférase en présence du composé de test comparée à un témoin, est indicative d'un composé qui inhibe l'activité de l'acétyl transférase.

**32.** Méthode selon la revendication 7, dans laquelle un composé qui augmente l'activité de l'acétyl transférase est identifié.

**33.** Méthode selon la revendication 32, dans laquelle une augmentation dans la valeur de polarisation de fluorescence du regroupement de substrats peptidiques à la mise en contact avec l'acétyl transférase en présence du composé de test comparée à un témoin, est indicative d'un composé qui augmente l'activité de l'acétyl transférase.

**34.** Méthode de détermination de l'activité d'une enzyme qui module l'acétylation d'un substrat peptidique, comprenant:

mettre un regroupement de substrats peptidiques en contact avec une enzyme qui module l'acétylation, où les membres dudit regroupement de substrats peptidiques comprennent un fluorophore et au moins un résidu de lysine ou un résidu de lysine acétylée;
mettre le regroupement de substrats peptidiques en contact avec un réactif de clivage qui clive les membres du regroupement de substrats peptidiques ayant des résidus de lysine non acétylée; et
déterminer la valeur de polarisation de fluorescence du regroupement de substrats peptidiques, où un changement dans la valeur de polarisation de fluorescence du regroupement de substrats peptidiques, est indicatif de l'activité de l'enzyme qui module l'acétylation.

**35.** Méthode d'identification d'un composé qui module l'activité d'une enzyme qui module l'acétylation d'un substrat peptidique, comprenant:

mettre un regroupement de substrats peptidiques en contact avec une enzyme qui module l'acétylation en présence d'un composé de test, où les membres dudit regroupement de substrats peptidiques comprennent un fluorophore et au moins un résidu de lysine ou un résidu de lysine acétylée;
mettre le regroupement de substrats peptidiques en contact avec un agent de clivage qui clive les membres du regroupement de substrats peptidiques ayant des résidus de lysine non acétylée; et
déterminer la valeur de polarisation de fluorescence du regroupement de substrats peptidiques, où un changement dans la valeur de polarisation de fluorescence du regroupement de substrats peptidiques en présence d'un composé de test, est indicatif d'un composé qui module l'activité de enzyme qui module l'acétylation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0214543 A
- US 4683195 A, Mullis
- US 5223409 A
- US 5976813 A, Beutel


### Non-patent literature cited in the description


- **Gray ; Ekstrom.** *Exper. Cell Res.,* 2001, vol. 262, 75-83
- **Zhou et al.** *Proc. Natl. Acad. Sci. USA,* 2001, vol. 98, 10572-10577
- **Kao et al.** *J. Biol. Chem.,* 2002, vol. 277, 187-193
- **Gao et al.** *J. Biol. Chem.,* 2002, vol. 277, 25748-25755
- **Sterner ; Berger.** *Microbiol. Mol. Biol. Rev.,* 2000, vol. 64, 435-459
- **Kramer et al.** *Trends Endocrinol. Metab.,* 2001, vol. 12, 294-300
- **Ait-Si-Ali et al.** *Nucleic Acids Res.,* 1998, vol. 26, 3869-3870
- **Tanner et al.** *J. Biol. Chem.,* 1999, vol. 274, 18157-18160
- **Wynne Aheme et al.** *Methods,* 2002, vol. 26, 245-53
- **Kim et al.** *Anal. Biochem.,* 2000, vol. 280, 308-314
- **Inoue ; Fujimoto.** *Biochim. Biophys. Acta,* 1970, vol. 220, 307-316
- **Nare et al.** *Anal. Biochem.,* 1999, vol. 267, 390-396
- **Hoffmann et al.** *Nucleic Acids Res.,* 1999, vol. 27, 2057-8
- **Hoffmann et al.** *Bioconjug Chem.,* 2001, vol. 12, 51-5
- **Hoffmann et al.** *Arch Pharm (Weinheim),* 2001, vol. 334, 248-52
- **Frey et al.** *224th National Meeting of the American Chemical Society,* August 2002
- **Marcotte et al.** *Anal. Biochem.,* 2004, vol. 332, 90
- **Wegener.** *Analytical Biochemistry,* 2003, vol. 321, 202-208
- **Marcotte.** *Analytical Biochemistry,* 2004, vol. 332, 90-99
- **Sambrook ; Fritsch ; Maniatis.** Molecular Cloning A Laboratory Manual. Cold Spring Harbor Laboratory Press, 1989
- DNA Cloning. 1985, vol. I, II
- Oligonucleotide Synthesis. 1984
- Nucleic Acid Hybridization. 1984
- Transcription And Translation. 1984
- Culture Of Animal Cells. Alan R. Liss, Inc, 1987
- Immobilized Cells And Enzymes. IRL Press, 1986
- **B. Perbal.** *A Practical Guide To Molecular Cloning,* 1984
- Methods In Enzymology. Academic Press, Inc,
- Gene Transfer Vectors For Mammalian Cells. Cold Spring Harbor Laboratory, 1987
- Methods In Enzymology. vol. 154, 155
- Immunochemical Methods In Cell And Molecular Biology. Academic Press, 1987
- Handbook Of Experimental Immunology. 1986, vol. I-IV
- Manipulating the Mouse Embryo. Cold Spring Harbor Laboratory Press, 1986
- Methods in Enzymology, vol. 266: Computer Methods for Macromolecular Sequence Analysis. Academic Press, Inc., a division of Harcourt Brace & Co, 1996, vol. 266
- *Meth. Mol. Biol.,* 1997, vol. 70, 173-187
- **Sterner ; Berger.** *Microbiol. Mol. Biol. Rev.,* 2000, vol. 64, 453-459
- **Brachmann et al.** *Genes Dev.,* 1995, vol. 9, 2888
- **Frye et al.** *BBRC,* 1999, vol. 260, 273
- **Lakowicz.** Principles of Fluorescence Spectroscopy. Plenum Press, 1983
- **Kumke et al.** *Anal. Chem.,* 1995, vol. 67, 3945-3951
- **Devlin et al.** *Clin. Chem.,* 1993, vol. 39, 1939-1943
- **Kaeberlein et al.** *JBC,* 2005, vol. 280 (17), 17038
- **McDonagh et al.** *Methods,* 2005, vol. 36, 346
- **Lam.** *Anticancer Drug Des.,* 1997, vol. 12, 145
- **DeWitt et al.** *Proc. Natl. Acad. Sci. U.S.A.,* 1993, vol. 90, 6909
- **Erb et al.** *Proc. Natl. Acad. Sci. U.S.A.,* 1994, vol. 91, 11422

- **Zuckermann et al.** *J Med Chem.,* 1994, vol. 37, 2678
- **Cho et al.** *Science,* 1993, vol. 261, 1303
- **Carell et al.** *Angew. Chem. Int. Ed Engl.,* 1994, vol. 33, 2059
- **Carell et al.** *Angew. Chem. Int. Ed. Engl.,* vol. 33, 2061
- **Gallop et al.** *J. Med Chem.,* 1994, vol. 37, 1233
- **Houghten.** *BioTechniques,* 1992, vol. 13, 412-421
- **Lam.** *Nature,* 1991, vol. 354, 82-84
- **Fodor.** *Nature,* 1993, vol. 364, 555-556
- **Cull et al.** *Proc. Natl. Acad Sci. U.S.A.,* 1992, vol. 89, 1865-1869
- **Scott ; Smith.** *Science,* 1990, vol. 249, 386-390
- **Devlin.** *Science,* 1990, vol. 249, 404-406
- **Cwirla et al.** *Proc. Natl. Acad. Sci.,* 1990, vol. 97, 6378-6382
- **Felici.** *J. Mol. Biol.,* 1991, vol. 222, 301-310
- **Jayawickreme et al.** *Proc. Natl. Acad. Sci. U.S.A.,* 1994, vol. 19, 1614-18
- **Chelsky.** Strategies for Screening Combinatorial Libraries: Novel and Traditional Approaches. *First Annual Conference of The Society for Biomolecular Screening in Philadelphia,* 07 November 1995
- **Salmon et al.** *Molecular Diversity,* 1996, vol. 2, 57-63